# EUROPEAN PATENT APPLICATION

(11) **EP 4 194 542 A1**
(43) Date of publication of application: **14.06.2023**
(21) Application number: 21306719.2
(22) Date of filing: 07.12.2021
(51) Int. Cl.: C12M 3/06, C12M 1/00, C12M 1/26, C12M 1/34, C12M 1/36, B01L 3/00, G01N 1/18

(54) **APPARATUS FOR FEEDING A LIQUID MEDIUM TO A FLUIDIC SYSTEM**

(71) Applicant: Fluigent, 94270 Le Kremlin-Bicêtre (FR)
(72) Inventor: CESAR, William, Paris (FR)
(74) Representative: Bandpay & Greuter

(57) **Abstract**

The present invention relates to an apparatus (100) for feeding a fluidic system (110) with liquid medium, the apparatus (100) comprising:
- a recirculation input reservoir (120), an injection reservoir (130), a recirculation output reservoir (140) and a sampling reservoir (150), each reservoir comprising a gas inlet (160), and a liquid inlet (170) or liquid outlet (165) equipped with a check valve (175);
- a refill channel (185) equipped with a check valve (186) from the input reservoir (120) to the recirculation output reservoir (140);
- a pressure control unit (190) comprising gas lines connected to the gas inlets (160) of the reservoirs and configured to control gas pressure in the reservoirs;
- the apparatus being configured to be connected to an inlet and an outlet of the fluidic system (110).

## Description

### TECHNICAL FIELD

The present invention relates to an apparatus for providing a liquid medium to a fluidic system. The invention also relates to a method of controlling flow rate of a liquid medium in a fluidic system. More specifically, the invention proposes an apparatus able to control pressure in a reservoir in order to provide and to control flow rate of a liquid medium in a fluidic system, with a performance, cost and simplicity superior to previous art.

### TECHNICAL BACKGROUND

Microfluidics has many applications amongst which is cell biology, and in particular the application of a so-called "Organ-On-A-Chip" (OOAC). Such an application consists in cultivating living cells of particular organs inside a microfluidics chip to grow functional tissues. In order to get the most accurate organ model, it is necessary to mimic the conditions cells undergo for *in vivo* growth. Such conditions may include temperature (e.g., 37°C), concentration of various chemicals (e.g., concentration of a dissolved gas, nutrients, proteins, hormones, etc.), or amount of mechanical stress. This application is useful for example for drug screening or testing. In this case, one or more drugs are put in contact with the cells and the medium is sampled to analyze what is rejected by the cells.

During cell culture, like in the body (e.g., human body), cells need nutrients and other chemicals in order to develop. In cell culture techniques, those factors are generally delivered by the so called "culture medium", a liquid that comes directly in contact with the cells and bring what they need and collect what they reject. There are several known methods to perform such a fluid recirculation such as, for instance, the use of peristaltic pumps. However, such methods are low responsive and provide poor flow stability. In addition, injecting or sampling lines in combination with such pumps requires the use of active valves to perform flow direction, which requires external actuation (electrical power or the manual strength of a researcher, for example).

As is known in the art, some culture methods use culture medium as a bath in which cells are simply immersed. However, these methods lack accuracy as, in these static conditions, cells do not undergo the same level of shear stress as *in vivo.* Also, using an inappropriate amount (e.g., too large or too small amount) of liquid medium in the bath tends to dilute (when the amount of liquid is too large), or on the contrary accumulate (when the amount of liquid is too large) chemicals released by the cells, so that these cells are not in a realistic "chemical environment".

In order to make the cultivation conditions closer to what happens in a normal tissue (e.g., in the human body) the liquid flow in an OOAC needs to satisfy some criteria, and should in particular be:
- Unidirectional: The stream of blood in blood vessels flows in one direction, due to the pumping mechanism of the heart. It has been shown that some cells, like endothelial cells, develop abnormally if the flow is not unidirectional (e.g., bidirectional).
- Stress-scaled: The flow rate should be adequately scaled (i.e., controlled) so that the cultured cells undergo a reasonable amount of shear stress. If the cells have a too low or too high shear stress (due to a too low or too high flow rate, respectively) at their membrane, they may not develop correctly.
- Volume-scaled : If the recirculated volume is too large, the chemicals released by the cells get too diluted and the cells do not grow in a "realistic" environment. On the contrary, if the recirculated volume is too low, the liquid gets saturated with "cell wastes" and/or gets depleted in nutrients or oxygen. It is thus important to be able to add fresh medium or remove old medium.

Document US 2020/181555 discloses an assembly, comprising an inlet reservoir and an outlet reservoir connected by a shortcut channel, said shortcut channel containing a valve, said inlet and outlet reservoirs in fluidic communication with a microfluidic device, said inlet reservoir comprising fluid, said microfluidic device comprising inlet and outlet ports. The document generally relates to microfluidic platforms or "chips" for testing and conducting experiments on the International Space Station.

Satoh et al., "A pneumatic pressure-driven multi-throughput microfluidic circulation culture system", Lab on a Chip 16: 2339-2348 (2016) discloses a pneumatic pressure-driven microfluidic device capable of multi-throughput medium circulation culture. The microfluidic device contains three independent circulation culture units, in which human umbilical vein endothelial cells (HUVECs) were cultured under physiological shear stress induced by circulation of the medium. Circulation of the medium in the three culture units was generated by programmed sequentially applied pressure from two pressure-control lines.

Other microfluidic devices are disclosed in: Li et al., "Squeeze-chip: a finger-controlled microfluidic flow network device and its application to biochemical assays", Lab on a Chip 12:1587-1590 (2012); Yang et al., "Pumpless microfluidic devices for generating healthy and diseased endothelia", Lab on a Chip 19:3212-3219 (2019); Reyes et al. "Micro Total Analysis Systems. 1. Introduction, Theory, and Technology", Anal. Chem. 74:2623-2636 (2002); US 7,223,363; US 2005/0180891; and WO 2008/101196.

The above documents do not solve the abovementioned criteria of the flowrate and/or do not provide a solution for injection and/or sampling. There is thus a need for an apparatus for providing a liquid medium to a fluidic system and in particular when the fluidic system is an OOAC.

### SUMMARY OF THE INVENTION

The invention relates to the following items.

Item 1. An apparatus for feeding a fluidic system with liquid medium, the apparatus comprising:
- a recirculation input reservoir and an injection reservoir, each comprising:
   ∘ at least one gas inlet, and
   ∘ at least one liquid outlet equipped with a check valve configured to allow a flow of liquid out of the reservoir;
- a recirculation output reservoir and a sampling reservoir each comprising:
   ∘ at least one gas inlet, and
   ∘ at least one liquid inlet equipped with a check valve configured to allow a flow of liquid into the reservoir;
- a refill channel fluidically connecting at least the recirculation input reservoir and the recirculation output reservoir, said channel being equipped with at least one check valve configured to allow a flow of liquid medium from the recirculation output reservoir to the recirculation input reservoir;
- a pressure control unit comprising gas lines connected to the gas inlets of the recirculation input reservoir, injection reservoir, recirculation output reservoir, and sampling reservoir and configured to control gas pressure in the recirculation input reservoir, injection reservoir, recirculation output reservoir, and sampling reservoir; and
- a first connection and a second connection each configured to be respectively connected to an inlet and to an outlet of the fluidic system;
wherein
- the recirculation input reservoir and the injection reservoir are configured for being fluidically connected to the fluidic system via the first connection; and
- the recirculation output reservoir and the sampling reservoir are configured for being fluidically connected to the fluidic system via the second connection.

In some variations of item 1, which may be present individually or in combination:
- The apparatus may comprise an adapter allowing the fluid connection of the apparatus to the fluidic system. The adapter may comprise one or more channels configured to provide hydrodynamic resistance.
- The apparatus may comprise a flowmeter on the first connection and/or on the second connection.
- There may be a plurality of injection reservoirs as defined above, such as two, or three, or four or more than four injection reservoirs.
- There may be a plurality of sampling reservoirs as defined above, such as two, or three, or four or more than four sampling reservoirs.
- There may be a plurality of injection reservoirs and a plurality of sampling reservoirs as defined above, such as two, or three, or four or more than four injection reservoirs and two, or three, or four or more than four sampling reservoirs.
- There may be a plurality of recirculation input reservoirs as defined above, such as two, or more than two recirculation input reservoirs.
- There may be a plurality of recirculation output reservoirs as defined above, such as two or more than two recirculation output reservoirs.
- There may be a plurality of recirculation input reservoirs and a plurality of recirculation output reservoirs as defined above, such as two or more than two recirculation input reservoirs and two or more than two recirculation output reservoirs.

Item 2. The apparatus according to item 1 including all variations, wherein the pressure control unit is configured to set gas pressure in the recirculation input reservoir, injection reservoir, recirculation output reservoir, and sampling reservoir at a value selected from a first pressure level and a second pressure level, at least one of the first pressure level and second pressure level being preferably the atmosphere pressure level.

Item 3. The apparatus according to any one of items 1 to 2 including all variations, wherein the pressure control unit comprises four selection valves on the gas lines respectively connected to the gas inlets of the recirculation input reservoir, injection reservoir, recirculation output reservoir and sampling reservoir.

Item 4. The apparatus according to item 3 including all variations, wherein each selection valve is fluidically connected to a first gas source at the first pressure level and to a second gas source at the second pressure level, and wherein preferably at least one of the first gas source and second gas source is the outlet of a pumping device. The other of the first gas source and second gas source is preferably the atmosphere.

Item 5. The apparatus according to any one of items 3 or 4 including all variations, wherein each selection valve is a three ways - two positions valve.

Item 6. The apparatus according to any one of items 1 to 5 including all variations, wherein each of the recirculation input reservoir, injection reservoir, recirculation output reservoir, and sampling reservoir is equipped with one or more liquid level sensor.

Item 7. The apparatus according to item 6 including all variations, wherein each liquid level sensor is configured to detect at least if a liquid level in the reservoir is above or below a threshold level.

Item 8. The apparatus according to any one of items 1 to 7 including all variations, comprising a cartridge and a manifold, the cartridge being configured to be removably mounted on the manifold, wherein
- the recirculation input reservoir, injection reservoir, recirculation output reservoir, and sampling reservoir are in the cartridge; and
- the pressure control unit is in the manifold.

Item 9. The apparatus according to item 8 including all variations, wherein:
- the manifold further comprises a printed circuit board (PCB) embedding comprising a plurality of magnetic coils, each magnetic coil being positioned so as to generate a magnetic field in a respective reservoir when the cartridge is mounted on the manifold; and/or
- the first connection and/or the second connection comprises a channel, a portion of which is in the cartridge and a portion of which is in the manifold, the portion in the manifold comprising a flowmeter; and/or
- the manifold comprises a plurality of liquid level sensors, each liquid level sensor positioned so as to detect a liquid level in a respective reservoir when the cartridge is mounted on the manifold.

In some variations of item 8 or item 9, which may be present individually or in combination:
- The apparatus may comprise a housing for receiving the manifold and the cartridge.
- The manifold may be permanently mounted within the housing whereas the cartridge may be removably mounted onto the manifold.
- The housing may comprise a cap (or lid) and a chassis.
- The housing cap may have an open position and a closed position and be configured to at least partially enclose the cartridge when it is mounted onto the manifold.
- The chassis may comprise a holder for the fluidic system.
- The housing may comprise a locking mechanism configured for locking the cartridge onto the manifold.
- In particular the housing cap may be configured to lock onto the chassis.
- The housing cap may comprise holes adapted to receive the respective reservoirs.
- The liquid level sensors, if present, may be provided on respective PCBs. One or more such PCBs may be grouped in respective level sensor casings fixed on the manifold. In some examples, a spring, such as a Y-spring, may be provided in each casing, so that the liquid level sensors are biased into contact with an outer surface of a respective reservoir, when the cartridge is mounted on the manifold.

Item 10. An assembly comprising the apparatus of any one of items 1 to 9 including all variations, and a fluidic system, the fluidic system being fluidically connected to the first connection and the second connection of the apparatus, preferably via an adapter.

Item 11. A method of feeding a fluidic system with liquid medium, wherein said fluidic system is fluidically connected to the first connection and the second connection of the apparatus according to any of items 1 to 10 including all variations, the method comprising supplying liquid medium to the fluidic system via the first connection and collecting liquid medium from the fluidic system via the second connection.

Item 12. The method according to item 11 including all variations, comprising
- a perfusing step, wherein the gas pressure in the reservoirs is adjusted so as to expel liquid medium from the recirculation input reservoir to the fluidic system via the first connection, and to collect liquid medium from the fluidic system to the recirculation output reservoir via the second connection; and/or
- a refilling step, wherein the gas pressure in the reservoirs is adjusted so as to make liquid medium flow from the recirculation output reservoir to the recirculation input reservoir via the refill channel.

Item 13. The method according to any one of items 11 or 12 including all variations, comprising:
- an injection step, wherein the gas pressure in the reservoirs is adjusted so as to expel liquid medium from the injection reservoir to the fluidic system via the first connection; and/or
- a sampling step, wherein the gas pressure in the reservoirs is adjusted so as to collect liquid medium from the fluidic system via the second connection to the sampling reservoir.

Item 14. The method according to any one of items 11 to 13 including all variations, wherein the liquid medium is a culture medium and wherein the fluidic system comprises living cells.

Item 15. A non-transitory computer readable storage medium having stored thereon instructions that, when executed, cause at least one control unit to carry out the method of any one of items 11 to 14 including all variations.

Alternatively, the apparatus may comprise at least one injection reservoir but no sampling reservoir as defined above. Accordingly, the invention also relates to the following item:
Item 16. An apparatus for feeding a fluidic system with liquid medium, the apparatus comprising:
   - a recirculation input reservoir and an injection reservoir, each comprising:
      ∘ at least one gas inlet, and
      ∘ at least one liquid outlet equipped with a check valve configured to allow a flow of liquid out of the reservoir;
   - a recirculation output reservoir comprising:
      ∘ at least one gas inlet, and
      ∘ at least one liquid inlet equipped with a check valve configured to allow a flow of liquid into the reservoir;
   - a refill channel fluidically connecting at least the recirculation input reservoir and the recirculation output reservoir, said channel being equipped with at least one check valve configured to allow a flow of liquid medium from the recirculation output reservoir to the recirculation input reservoir;
   - a pressure control unit comprising gas lines connected to the gas inlets of the recirculation input reservoir, injection reservoir and recirculation output reservoir and configured to control gas pressure in the recirculation input reservoir, injection reservoir and recirculation output reservoir; and
   - a first connection and a second connection each configured to be respectively connected to an inlet and to an outlet of the fluidic system;
   wherein
   - the recirculation input reservoir and the injection reservoir are configured for being fluidically connected to the fluidic system via the first connection; and
   - the recirculation output reservoir is configured for being fluidically connected to the fluidic system via the second connection.

Alternatively, the apparatus may comprise at least one sampling reservoir but no injection reservoir as defined above. Accordingly, the invention also relates to the following item:
Item 17. An apparatus for feeding a fluidic system with liquid medium, the apparatus comprising:
   - a recirculation input reservoir comprising:
      ∘ at least one gas inlet, and
      ∘ at least one liquid outlet equipped with a check valve configured to allow a flow of liquid out of the reservoir;
   - a recirculation output reservoir and a sampling reservoir each comprising:
      ∘ at least one gas inlet, and
      ∘ at least one liquid inlet equipped with a check valve configured to allow a flow of liquid into the reservoir;
   - a refill channel fluidically connecting at least the recirculation input reservoir and the recirculation output reservoir, said channel being equipped with at least one check valve configured to allow a flow of liquid medium from the recirculation output reservoir to the recirculation input reservoir;
   - a pressure control unit comprising gas lines connected to the gas inlets of the recirculation input reservoir, recirculation output reservoir, and sampling reservoir and configured to control gas pressure in the recirculation input reservoir, recirculation output reservoir, and sampling reservoir; and
   - a first connection and a second connection each configured to be respectively connected to an inlet and to an outlet of the fluidic system;
   wherein
   - the recirculation input reservoir is configured for being fluidically connected to the fluidic system via the first connection; and
   - the recirculation output reservoir and the sampling reservoir are configured for being fluidically connected to the fluidic system via the second connection.

The apparatuses of items 16 and 17 may also have the additional features described in, or may also be implemented in the manner described in, any of items 2 to 15 including all variations, except that any reference to the sampling reservoir, respectively to the injection reservoir, should then be considered as being omitted in these items 2 to 15.

The invention also relates to the following group of items.

Item 18. An apparatus for feeding a fluidic system with liquid medium, the apparatus comprising a cartridge and a manifold, the cartridge being configured to be removably mounted on the manifold, the apparatus comprising:
- a recirculation input reservoir positioned in the cartridge, comprising:
   ∘ at least one gas inlet, and
   ∘ at least one liquid outlet equipped with a check valve configured to allow a flow of liquid out of the reservoir;
- a recirculation output reservoir positioned in the cartridge, comprising:
   ∘ at least one gas inlet, and
   ∘ at least one liquid inlet equipped with a check valve configured to allow a flow of liquid into the reservoir;
- a refill channel fluidically connecting at least the recirculation input reservoir and the recirculation output reservoir, said channel being equipped with at least one check valve configured to allow a flow of liquid medium from the recirculation output reservoir to the recirculation input reservoir;
- a pressure control unit in the manifold, comprising gas lines connected to the gas inlets of the recirculation input reservoir and recirculation output reservoir and configured to control gas pressure in the recirculation input reservoir and recirculation output reservoir; and
- a first connection and a second connection each configured to be respectively connected to an inlet and to an outlet of the fluidic system;
wherein
- the recirculation input reservoir is configured for being fluidically connected to the fluidic system via the first connection; and
- the recirculation output reservoir is configured for being fluidically connected to the fluidic system via the second connection.

Item 19. The apparatus according to item 18, wherein:
- the manifold further comprises a printed circuit board (PCB) embedding comprising a plurality of magnetic coils, each magnetic coil being positioned so as to generate a magnetic field in a respective reservoir when the cartridge is mounted on the manifold; and/or
- the first connection and/or the second connection comprises a channel, a portion of which is in the cartridge and a portion of which is in the manifold, the portion in the manifold comprising a flowmeter; and/or
- the manifold comprises a plurality of liquid level sensors, each liquid level sensor positioned so as detect a liquid level in a respective reservoir when the cartridge is mounted on the manifold.

In some variations of item 18 or item 19, which may be present individually or in combination:
- The apparatus may comprise a housing for receiving the manifold and the cartridge.
- The manifold may be permanently mounted within the housing whereas the cartridge may be removably mounted onto the manifold.
- The housing may comprise a cap (or lid) and a chassis.
- The housing cap may have an open position and a closed position and be configured to enclose the cartridge when it is mounted onto the manifold.
- The chassis may comprise a holder for the fluidic system.
- The housing may comprise a locking mechanism configured for locking the cartridge onto the manifold.
- In particular the housing cap may be configured to lock onto the chassis.
- The housing cap may comprise holes adapted to receive the respective reservoirs.
- The liquid level sensors, if present, may be provided on respective PCBs. One or more such PCBs may be grouped in respective level sensor casings fixed on the manifold. In some examples, a spring, such as a Y-spring, may be provided in each casing, so that the liquid level sensors are biased into contact with a surface of a respective reservoir, when the cartridge is mounted on the manifold.

Item 20. An assembly comprising the apparatus of any one of items 18 to 19 including all variations, and a fluidic system, the fluidic system being fluidically connected to the first connection and the second connection of the apparatus, preferably via an adapter.

Item 21. A method of feeding a fluidic system with liquid medium, wherein said fluidic system is fluidically connected to the first connection and the second connection of the apparatus according to any of items 18 to 19 including all variations, the method comprising supplying liquid medium to the fluidic system via the first connection and collecting liquid medium from the fluidic system via the second connection.

Item 22. The method according to item 21 including all variations, comprising
- a perfusing step, wherein the gas pressure in the reservoirs is adjusted so as to expel liquid medium from the recirculation input reservoir to the fluidic system via the first connection, and to collect liquid medium from the fluidic system to the recirculation output reservoir via the second connection; and/or
- a refilling step, wherein the gas pressure in the reservoirs is adjusted so as to make liquid medium flow from the recirculation output reservoir to the recirculation input reservoir via the refill channel.

Item 23. The method according to any one of items 21 to 22 including all variations, wherein the liquid medium is a culture medium and wherein the fluidic system comprises living cells.

Item 24. A non-transitory computer readable storage medium having stored thereon instructions that, when executed, cause at least one control unit to carry out the method of any one of items 21 to 23 including all variations.

In items 18 to 24, at least one sampling reservoir or at least one injection reservoir may additionally be present, as described above in connection with items 16 and 17, including all variations. Or both at least one sampling reservoir and at least one injection reservoir may additionally be present, as described above in connection with items 1 to 15, including all variations. Whether or not a sampling reservoir and/or an injection reservoir is present, all features disclosed in connection with items 1 to 15, including all variations, apply similarly to items 18 to 24 - except that any reference to an injection reservoir and/or to a sampling reservoir should be omitted, if such reservoir is not present.

The invention also relates to the following group of items.

Item 25. An apparatus for feeding a fluidic system with liquid medium, the apparatus comprising:
- a recirculation input reservoir comprising:
   ∘ at least one gas inlet, and
   ∘ at least one liquid outlet equipped with a check valve configured to allow a flow of liquid out of the reservoir;
- a recirculation output reservoir comprising:
   ∘ at least one gas inlet, and
   ∘ at least one liquid inlet equipped with a check valve configured to allow a flow of liquid into the reservoir;
- a refill channel fluidically connecting at least the recirculation input reservoir and the recirculation output reservoir, said channel being equipped with at least one check valve configured to allow a flow of liquid medium from the recirculation output reservoir to the recirculation input reservoir;
- a pressure control unit comprising gas lines connected to the gas inlets of the recirculation input reservoir and recirculation output reservoir and configured to control gas pressure in the recirculation input reservoir and recirculation output reservoir; and
- a first connection and a second connection each configured to be respectively connected to an inlet and to an outlet of the fluidic system;
wherein
- the recirculation input reservoir is configured for being fluidically connected to the fluidic system via the first connection; and
- the recirculation output reservoir is configured for being fluidically connected to the fluidic system via the second connection;
wherein each reservoir is equipped with one or more liquid level sensors.

Item 26. The apparatus according to item 25, wherein each liquid level sensor is configured to detect at least if a liquid level in the reservoir is above or below a threshold level.

Item 27. The apparatus according to item 26, comprising a cartridge and a manifold, the cartridge being configured to be removably mounted on the manifold, wherein
- the recirculation input reservoir and recirculation output reservoir are in the cartridge; and
- the pressure control unit is in the manifold.

Item 28. The apparatus according to item 27, wherein:
- the manifold further comprises a printed circuit board (PCB) embedding comprising a plurality of magnetic coils, each magnetic coil being positioned so as to generate a magnetic field in a respective reservoir when the cartridge is mounted on the manifold; and/or
- the first connection and/or the second connection comprises a channel, a portion of which is in the cartridge and a portion of which is in the manifold, the portion in the manifold comprising a flowmeter.

In some variations of item 28, which may be present individually or in combination:
- The apparatus may comprise a housing for receiving the manifold and the cartridge.
- The manifold may be permanently mounted within the housing whereas the cartridge may be removably mounted onto the manifold.
- The housing may comprise a cap (or lid) and a chassis.
- The housing cap may have an open position and a closed position and be configured to enclose the cartridge when it is mounted onto the manifold.
- The chassis may comprise a holder for the fluidic system.
- The housing may comprise a locking mechanism configured for locking the cartridge onto the manifold.
- In particular the housing cap may be configured to lock onto the chassis.
- The housing cap may comprise holes adapted to receive the respective reservoirs.
- The liquid level sensors may be provided on respective PCBs. One or more such PCBs may be grouped in respective level sensor casings fixed on the manifold. In some examples, a spring, such as a Y-spring, may be provided in each casing, so that the liquid level sensors are biased into contact with a surface of a respective reservoir, when the cartridge is mounted on the manifold.

Item 29. An assembly comprising the apparatus of any one of items 25 to 28 including all variations, and a fluidic system, the fluidic system being fluidically connected to the first connection and the second connection of the apparatus, preferably via an adapter.

Item 30. A method of feeding a fluidic system with liquid medium, wherein said fluidic system is fluidically connected to the first connection and the second connection of the apparatus according to any of items 25 to 28 including all variations, the method comprising supplying liquid medium to the fluidic system via the first connection and collecting liquid medium from the fluidic system via the second connection.

Item 31. The method according to item 30 including all variations, comprising:
- a perfusing step, wherein the gas pressure in the reservoirs is adjusted so as to expel liquid medium from the recirculation input reservoir to the fluidic system via the first connection, and to collect liquid medium from the fluidic system to the recirculation output reservoir via the second connection; and/or
- a refilling step, wherein the gas pressure in the reservoirs is adjusted so as to make liquid medium flow from the recirculation output reservoir to the recirculation input reservoir via the refill channel.

Item 32. The method according to any one of items 30 to 31 including all variations, wherein the liquid medium is a culture medium and wherein the fluidic system comprises living cells.

Item 33. A non-transitory computer readable storage medium having stored thereon instructions that, when executed, cause at least one control unit to carry out the method of any one of items 30 to 32 including all variations.

In items 25 to 33, at least one sampling reservoir or at least one injection reservoir may additionally be present, as described above in connection with items 16 and 17, including all variations. Or both at least one sampling reservoir and at least one injection reservoir may additionally be present, as described above in connection with items 1 to 15, including all variations. Whether or not a sampling reservoir and/or an injection reservoir is present, all features disclosed in connection with items 1 to 15, including all variations, apply similarly to items 25 to 33 - except that any reference to an injection reservoir and/or to a sampling reservoir should be omitted, if such reservoir is not present.

Embodiments of the present invention make it possible to address the needs expressed above. In particular, the one or more embodiments provide an apparatus which makes it possible to supply liquid flow in a fluidic system such as an OOAC in a manner which is unidirectional, stress-scaled and volume-scaled; and which makes it possible to perform injection and/or sampling.

### BRIEF DESCRIPTION OF THE DRAWINGS

**Figures 1****, 2A-B** show schematic diagrams of a comparative apparatus, not according to the invention.
**Figure 3** schematically shows an example apparatus according to an embodiment of the invention.
**Figure 4A** shows a schematic view of a selection valve and **Figure 4B** shows a schematic example of a check valve.
**Figure 5A** shows an example fabricated apparatus according to an embodiment of the invention.
**Figure 5B** shows an exploded schematic view of another example fabricated apparatus according to an embodiment of the invention.
**Figures 6A****-C** schematically show a cartridge of the apparatus according to an embodiment.
**Figures 7A****-E** schematically show the manifold of the apparatus according to an embodiment.
**Figures 8A-E** are different views another example apparatus according to an embodiment of the invention.
**Figure 9** shows a schematical cross-section of an assembly according to one embodiment of the invention.
**Figure 10** shows global physical interconnection of the apparatus according to an embodiment of the invention.
**Figure 11** shows electrical interconnections of the apparatus according to an embodiment of the invention.
**Figure 12** shows an example an elastomer check valve in the apparatus, according to one embodiment.
**Figures 13A-C** and **14** schematically show how the cartridge is locked and unlocked according to one embodiment.
**Figures 15A-B** show the mechanism of magnetic stirring in the reservoirs of the apparatus, according to one embodiment.
**Figures 16A-E** show the mechanism of fluid level detection in the apparatus, according to one embodiment.

### DESCRIPTION OF EMBODIMENTS

Embodiments of the invention will now be described in more detail without limitation in the following description.

### Apparatus for providing a liquid medium to a fluidic system

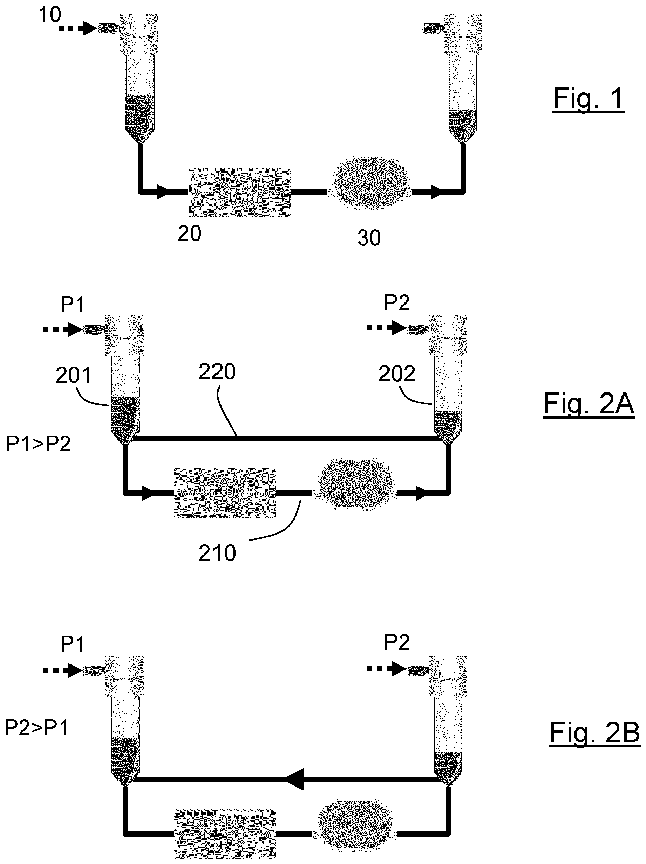

**Figures 1,** and **2A-B** show comparative assemblies in which two reservoirs are fluidically connected to a fluidic system, i.e., an OOAC to provide a flow of liquid to the OOAC.

According to the comparative assembly of **figure 1****,** a gas (e.g., air) 10 is pressurized above an input liquid reservoir air pressure so as to drive a flow by pushing the liquid inside a tubing on which the OOAC 20 is installed (in the arrow direction), thereby flowing it through the chip before collecting it in an output reservoir. A flowmeter 30 may also be used in the tubing, so that the input pressure is tuned using a regulation loop to regulate and reach the desired flowrate. However this method does not directly allow to perform continuous recirculation of liquid medium as the flow direction is always from the reservoir with the higher pressure to the one with the lower pressure, so that at some point one reservoir is empty and the flow must be stopped.

The comparative assembly of **figures 2A-2B** uses a "perfuse then refill" technique to solve the discontinuous recirculation issue by using a two-step method based on alternating two steps. The first step as depicted in **figure 2A** is called "perfusion" and consists in flowing the medium through the tubing, here called "perfusion line" 210, from an input reservoir 201 to an output reservoir 202. During this step, the pressure of the input reservoir 201 is higher than the pressure of the output reservoir 202. Optionally a flowmeter may be mounted on the perfusion line if flow regulation is needed. The second step is a step of "refilling" as depicted in **figure 2B****,** which consists in transferring the medium back from the output reservoir 202 to the input reservoir 201 through a dedicated "refill line" 220. In this technique, there is no flow in the chip during the refilling step. During this step, the pressure of the input reservoir 201 is lower than the pressure of the output reservoir 202.

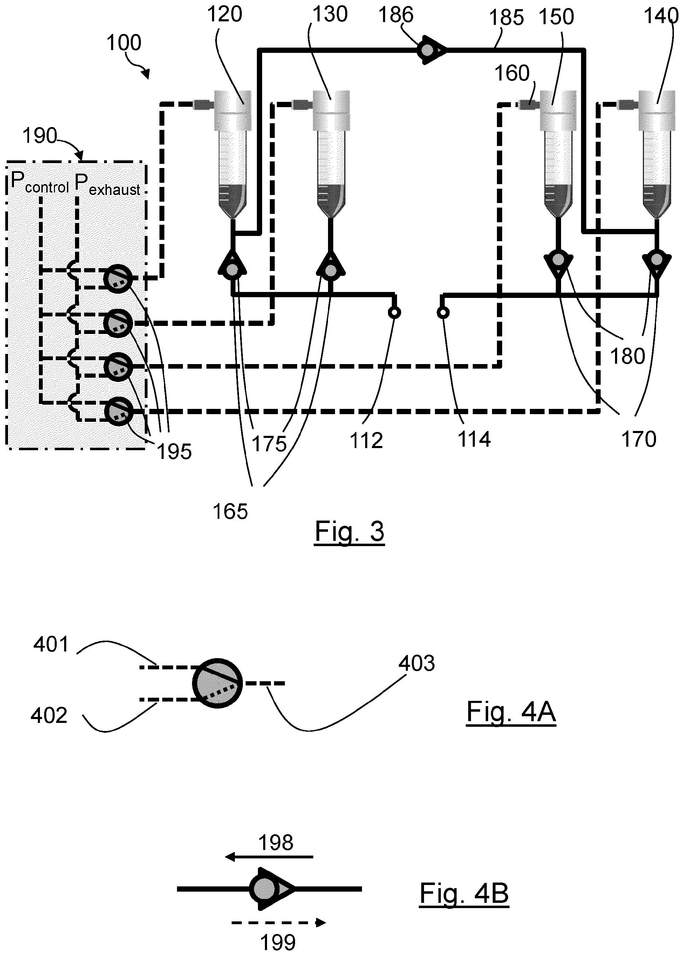

One or more embodiments of the invention relate to an apparatus 100 as depicted in **figure** 3 for providing a liquid medium to a fluidic system 110. A "fluidic system" designates a combination of one or more instruments associated to exert one or several tasks in relation to one or more fluids. By "instrument" is meant an integrated device that is able to perform at least one function without the addition of additional components other than components available in the operational environment, such as for instance an energy source, or consumables. A fluidic system may comprise at least one channel but optionally comprises other components. A fluidic system may comprise components which are fluidic in their nature and/or function. Fluidic systems may involve different levels of integration. For instance, they can be restricted to a single fluidic chip or component, integrating one or several functionalities. Fluidic systems used in the invention may also comprise other kinds of elements and components, such as pumps, valves, sensors, actuators, detectors, and many others known in the art, which are encompassed within the scope of the invention. In particular, fluidic systems may also be full instruments and comprise for instance any of holders, housings, power sources, control software and hardware, communication means, storage means, manipulation means, human-machine interfaces.

The fluidic system may notably be a microfluidic, millifluidic, or nanofluidic system or any combination thereof. By "millifluidic system" is meant a fluidic system in which the minimal channel dimension is of the order of 1-10 mm. By "microfluidic system" is meant a fluidic system in which the minimal channel dimension is of the order of 1 to less than 1000 µm. By "nanofluidic system" is meant a fluidic system in which the minimal channel dimension is of the order of less than 1 µm.

By "fluidic chip" or equivalently "chip", or equivalently "fluidic component", is meant an object comprising at least one channel, or at least one combination of channels. The channel or combination of channels are embedded at least in part in a matrix. Fluidic chips or devices may be microfluidic chips or devices, i.e., comprise at least one microchannel (channel having a minimum dimension of the order of 1 to less than 1000 µm). Fluidic chips or devices may be millifluidic chips or devices, i.e., comprise at least one millichannel (channel having a minimum dimension of the order of 1-10 mm). Fluidic chips or devices may be nanofluidic chips or devices, i.e., comprise at least one nanochannel (channel having a minimum dimension of less than 1 µm). Fluidic chips or devices may comprise any combination of millichannels, nanochannels or microchannels.

In particular examples of the method, the fluidic system may be or may comprise an Organ-On-A-Chip (OOAC).

The apparatus illustrated in **figure 3** comprises a recirculation input reservoir 120 and an injection reservoir 130 each comprising at least one gas inlet 160, and at least one liquid outlet 165 equipped with a check valve 175 configured to allow a flow of liquid out of the reservoir (outward check valve). The apparatus 100 further comprises a recirculation output reservoir 140 and a sampling reservoir 150 each comprising at least one gas inlet 160, and at least one liquid inlet 170 equipped with a check valve 180 configured to allow a flow of liquid into the reservoir (inward check valve). As mentioned above in the summary section, in alternate variations, the sampling reservoir may be absent; in other, alternate variations, the injection reservoir may be absent.

By check valve is meant a unidirectional valve, which allows the flow of fluid in only one direction 198 (and therefore prevents the flow of fluid in the opposite direction 199). An example of check valve is schematically illustrated in **figure 4B****.** By way of example, each check valve may be of a ball valve type, in which a ball blocks the channel when the fluid tends to flow in one direction and unblocks the channel when the fluid flows in the opposite direction.

The apparatus 100 further comprises a refill channel 185 fluidically connecting at least the recirculation input reservoir 120 and the recirculation output reservoir 140, said channel being equipped with at least one check valve 186 configured to allow a flow of liquid medium from the recirculation output reservoir 140 to the recirculation input reservoir 120.

The apparatus 100 further comprises a pressure control unit 190 comprising gas lines connected to the gas inlets 160 of the recirculation input reservoir, injection reservoir, recirculation output reservoir, and sampling reservoir and configured to control gas pressure in the recirculation input reservoir, injection reservoir, recirculation output reservoir, and sampling reservoir The pressure control unit 190 may comprise four selection valves 195 on the gas lines respectively connected to the gas inlets 160 of the recirculation input reservoir, injection reservoir, recirculation output reservoir and sampling reservoir.

The apparatus 100 further comprises a first connection 112 and a second connection 114 each configured to be connected to an inlet and to an outlet of the fluidic system 110, respectively. In the apparatus 100, the liquid outlets 165 of the recirculation input reservoir 120 and of the injection reservoir 130 are fluidically connected to the first connection 112. As a result, the recirculation input reservoir 120 is configured for being fluidically connected to the fluidic system 110 via the first connection 112 and the recirculation output reservoir 140 is configured for being fluidically connected to the fluidic system 110 via the second connection 114. The liquid inlets 170 of the recirculation output reservoir 140 and of the sampling reservoir 150 are fluidically connected to the second connection 114. As a result, the injection reservoir 130 is configured for being fluidically connected to the fluidic system 110 via the first connection 112; and the sampling reservoir 150 is configured for being fluidically connected to the fluidic system 110 via the second connection 114. The connection of the recirculation input reservoir 120 to the first connection 112 and the connection of the recirculation output reservoir 140 to the second connection 114 partially form a perfusing channel configured to allow a flow of liquid medium from the recirculation input reservoir 120 to the recirculation output reservoir 140. The perfusing channel thus comprises the first connection 112 and the second connection 114.

The check valves 175, 180, and 186 may provide a mechanism to block backflow during a perfusion of flow of liquid medium from the recirculation input reservoir 120 to the recirculation output reservoir 140 in the perfusing channel, and during a refill by a flow of liquid medium from the recirculation output reservoir 140 to the recirculation input reservoir 120 in the refill channel 185. Such a mechanism may block any flow in the refill channel 185 during perfusion, and blocks any flow in the perfusing channel during the refill.

The first connection 112 may be or may comprise a channel, as well as a port for fluidically connecting at least one inlet of the fluidic system to this channel. Similarly, the second connection 114 may be or may comprise a channel, as well as a port for fluidically connecting at least one outlet of the fluidic system to this channel. Optionally, the apparatus 100 may comprise at least one flowmeter (as discussed in more detail below) on the perfusing channel as well as channels leading to and from the flowmeter. Such channels are configured to be fluidically connected to the first connection 112, and/or to the second connection 114. The flowmeter may be used in order to measure and/or control the flow in the perfusing channel. Alternatively or additionally, the apparatus 100 may comprise at least one flowmeter on the refill channel 185 in order to measure and/or control the flow in said channel (not illustrated).

By A being *"fluidically connected"* to B, is meant that a fluid may flow between A and B, i.e., the fluid path between A and B is not fully closed. Two reservoirs may form "recirculation reservoir pair", namely the "recirculation input (RI) reservoir" and the "recirculation output (RO) reservoir"; the "injection reservoir" or "input (I) reservoir", and the "sampling reservoir" or "output (O) reservoir" are two further reservoirs, which may be present in the apparatus of the invention. Furthermore, the refill channel 185 connecting the two reservoirs of the recirculation reservoir pair is equivalently called "recirculation line" or "recirculation channel" as it provides the possibility of recirculating liquid from the reservoir with an inward check valve to the reservoir with an outward check valve.

The presence of an injection reservoir allows the injection of other fluids in the liquid medium fed to the fluidic system, for example a buffer solution or a drugcontaining solution. The presence of a sampling reservoir allows collecting a sample of the liquid medium for further analysis, for example chemical analysis.

In examples, the apparatus may comprise more than one recirculation reservoir pairs such that each of the input reservoir of each pair comprises at least one gas inlet 160, and at least one liquid outlet 165 equipped with a check valve 175 configured to allow a flow of liquid out of said reservoir, and each output reservoir of each pair comprises at least one gas inlet 160, and at least one liquid inlet 170 equipped with a check valve 180 configured to allow a flow of liquid into the reservoir.

The apparatus 100 may comprise more than one injection reservoir and/or more than one sampling reservoir. Each injection reservoir comprises at least one gas inlet 160, and at least one liquid outlet 165 equipped with a check valve 175 configured to allow a flow of liquid out of said reservoir, and may be fluidically connected to the first connection 112. Each sampling reservoir comprises at least one gas inlet 160, and at least one liquid inlet 170 equipped with a check valve 180 configured to allow a flow of liquid into the reservoir, and may be fluidically connected to the second connection 114.

In examples, the apparatus 100 may comprise more than four reservoirs. In such examples, the apparatus may comprise more than one recirculation reservoir pair each reservoir of each pair is connected to a respective check valve. More specifically, one of the reservoirs of each pair is equipped with a check valve configured to allow a flow inward to the reservoir while the other one is equipped with a check valve configured to allow a flow outward from the reservoir. Each pair is further connected by a recirculation line equipped with a check valve as discussed above. Further in such examples, the apparatus 100 may comprise more than one injection reservoir each equipped with an outward check valve, i.e., allowing a flow of fluid out of the reservoir. Further in such examples, the apparatus 100 may comprise more than one sampling reservoir each equipped with an inward check valve, i.e., allowing a flow of fluid into the reservoir.

In examples, the capacity of each of the reservoirs is from 0.5 mL to 50 mL, preferably from 1 to 10 mL, such as for example about 5 mL. In examples, all the reservoirs have a same capacity. If the capacity of the reservoirs is too small, level detection in the reservoirs may be difficult due to the relative size of the meniscus or in view of the necessary relative spacing between different level sensors.

### Structure of the apparatus

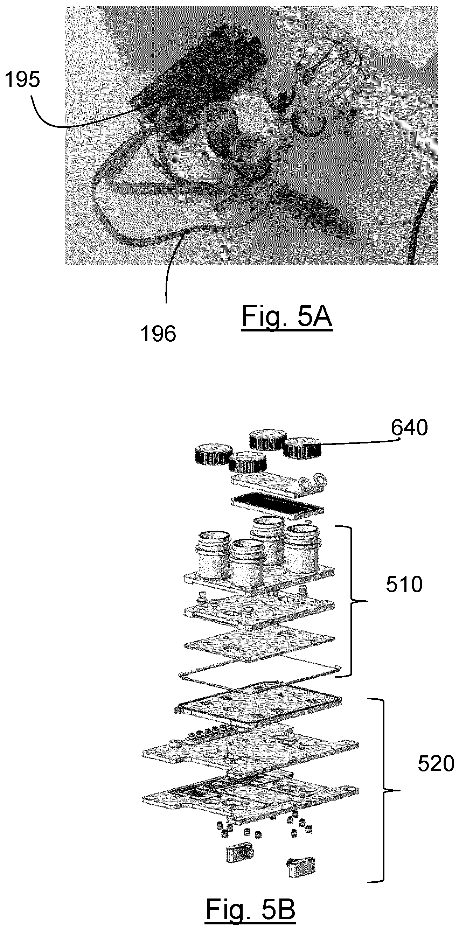

An example of apparatus 100 is presented in **figures 5A-B.** **Figure 5A** presents a fabricated example of apparatus and in particular presents a controller part 195 of the pressure control unit 190 and its electrical connections 196. The controller part of the pressure control unit may be configured to control functions of the pressure control unit, for example the controller part may comprise a non-transitory computer readable storage medium having stored thereon instructions for controlling said functions.

The apparatus 100 may have a modular construction, i.e., the apparatus may be made of different parts which may be mounted and/or dismounted depending on the need, for example so as to make any of the reservoirs disposable (for contamination reasons) or so as to make the connections to the fluidic system easier. **Figure 5B** presents an exploded view of such a modular apparatus. The apparatus 100 is made of different parts which when mounted altogether and properly sealed, make a single, integrated apparatus that allows medium recirculation, injection and sampling with a precise and stable flow control, thanks to pressure control through flowrate measurement and regulation.

In such examples, the apparatus 100 comprises a cartridge 510 and a manifold 520.

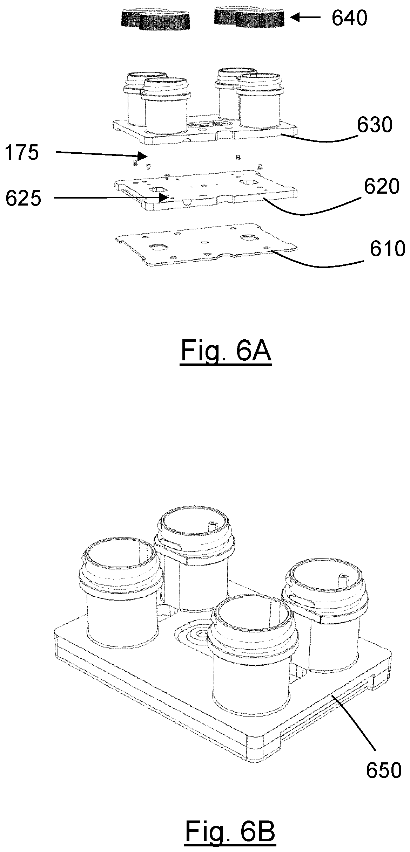

The mechanical and fluidic structure of the cartridge is now discussed in reference to **figures 6A-C.**

The cartridge may be configured to be removably mounted on the manifold. By "removably mounted" is meant that the cartridge and the manifold are unitary or permanently fixed, and may be detached from each other and reattached, for example the cartridge may be attached to the manifold by a detachable joint (e.g., bolt, screw, or a dent or groove 650 on one or more of the manifold or the cartridge). The recirculation input reservoir, injection reservoir, recirculation output reservoir, and sampling reservoir may be in the cartridge. The cartridge may comprise a frame. In some examples, the recirculation input reservoir, injection reservoir, recirculation output reservoir, and sampling reservoir may be an integral part of the cartridge, i.e., may be integrally formed with, or permanently fixed to the cartridge frame. Alternatively, the recirculation input reservoir, injection reservoir, reservoir, and sampling reservoir removably fixed to the frame of the cartridge. For example, the cartridge frame may comprise slots in which the recirculation input reservoir, injection reservoir, reservoir, and sampling reservoir may be engaged. The reservoirs may be mounted using connectors (comprising or consisting of proper seals or gaskets ensuring a connection between respective ports) to allow pressure to be set in the reservoirs and liquid to flow from or to the reservoirs.

**Figures 6A-B** present a mechanical structure of the cartridge. The cartridge 510 comprises three layers which are assembled together so as to form the frame, namely a bottom layer 610, a middle layer 620 and a top layer 630. The top layer 630 includes the reservoirs, i.e., the recirculation input reservoir 120, injection reservoir 130, recirculation output reservoir 140, and sampling 150 reservoir. The middle layer 620 comprises check-valve slots 625 (i.e., slots on which the check valves 175 are mounted). Each of the top layer 630 and the middle layer 620 further comprises channels, connecting the reservoirs to the check valves 175, to the manifold, and to the pressure control unit. The channels at least comprise the refill channel 185. The cartridge 510 may also comprise respective caps 640 for each of the reservoirs. Each cap 640 may be mounted on a respective reservoir in a pressure-tight (i.e., leak-tight) manner. The cap 640 may be designed for a pressure lower or equal to 1.5 bar. The cartridge may further have a locking feature, such as a groove 650, to lock (i.e., mount) the cartridge 510 to the manifold. This mechanism is discussed in more detail below.

In some examples, each check valve 175, 186 may be an elastomer check valve, e.g., a duckbill valve. These valves are passive valves and allow flow only in one direction. Examples of duckbill valves are presented in **figure 17**.

The cartridge 510 may be fabricated using multilayer bonding of plastic (molded or machined) parts. In particular, reservoir walls may be made of at least one of polycarbonate, polymethyl methacrylate (PMMA), polypropylene (PP), polyethylene (PE), polystyrene (PS), polyether ether ketone (PEEK), cyclic olefin copolymer (COC), and polyetherimide (PEI) materials like Ultem^{™}.

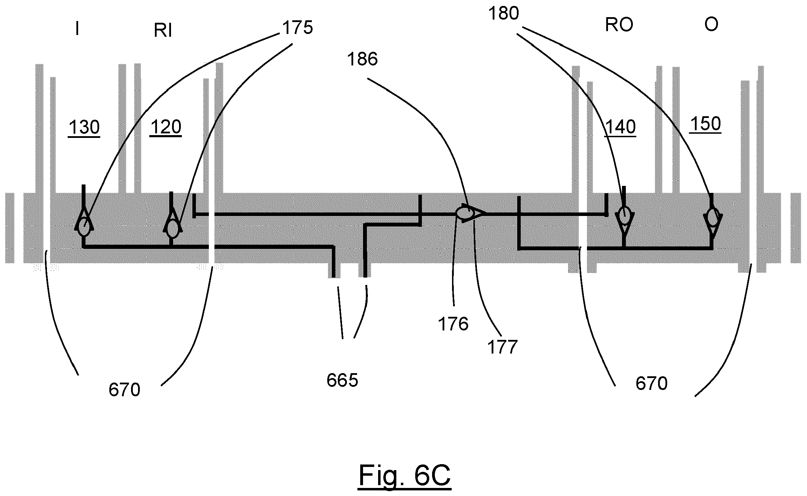

**Figures 6C** shows the fluidic structure of the cartridge 510. In such structure the refill channel 185 connects the RI and RO reservoirs. Further the first connection 112 fluidically connects the output of the RI and I reservoirs to a first port configured to be fluidically connected to an inlet of the fluidic system, and the second connection 114 fluidically connects the input of the RO and O reservoirs to a second port configured to be fluidically connected to an outlet of the fluidic system. The first port and the second port may be fluidically connected to the fluidic system via an adapter as discussed in detail below. As illustrated, the first connection 112 may be interrupted by two diversion ports 665 configured for being fluidically connected to the manifold. In other words, the first connection 112 may comprise one portion in the cartridge and another portion in the manifold (described in more detail below). Alternatively or additionally (not shown), the second connection 114 may be interrupted by two diversion ports 665 configured for being fluidically connected to the manifold. In other words, the second connection 114 may comprise one portion in the cartridge and another portion in the manifold (described in more detail below). The cartridge may also comprise gas line portions 670 fluidically connected to the respective reservoir via their respective gas inlets 160. Each of the check valves 175, 180, and 186 in **figure 6C** may be of the ball valve type, in which a ball 176 blocks the channel in the direction pointed by triangle 177.

Preferably, the liquid outlets 165 and liquid inlets 170 of each reservoir are positioned at the bottom of the respective reservoirs, whereas the gas inlets 160 are positioned away from the bottom, for example at the top, of the respective reservoirs.

The mechanical and fluidic structure of the manifold is now discussed.

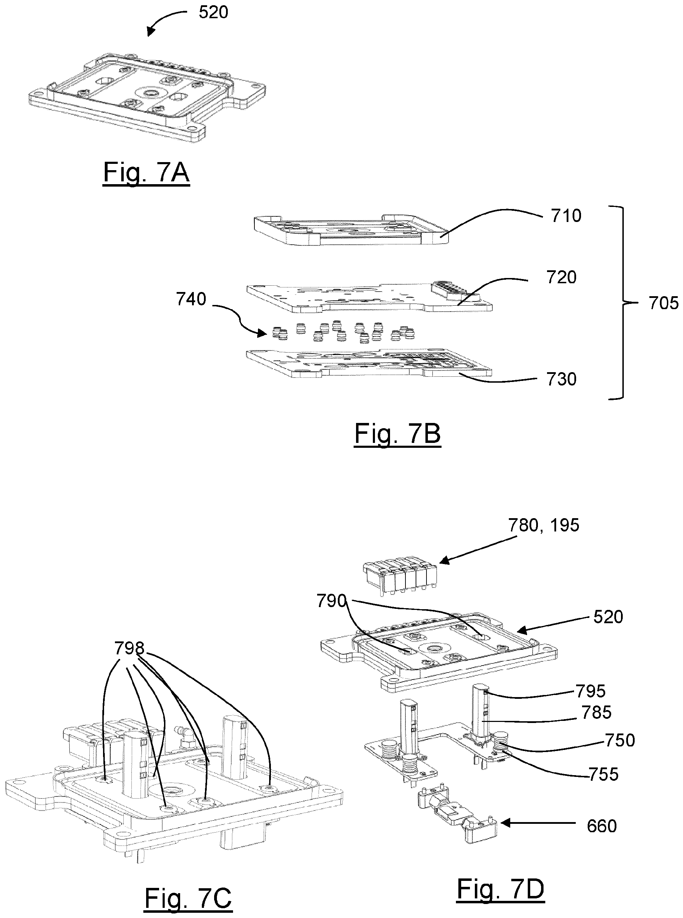

**Figures 7A-B** present an example of the manifold 520 when in a bare state, i.e., only the main structure of the manifold, in a schematic and exploded view, respectively. The manifold 520 may comprise a frame 705. This frame may comprise a top layer 710, a middle layer 720, and a bottom layer 730. The top layer 710 may be in contact with the cartridge 510 and be the layer on which the cartridge 510 is mounted. The manifold 520 may include the selection valves 195 of the pressure control unit 190, for example they may be mounted on the middle layer 720 of the manifold 520. Furthermore, the manifold 520 may include a flowmeter 660; and the manifold 520 may include one or more printed circuit boards (PCBs); the flowmeter 660 and the PCBs may be mounted on the bottom layer 730. Upon mounting the cartridge 510 on the manifold 520, the cartridge 510 and the manifold 520 are fluidically connected. The cartridge 510 and the manifold 520 may also be pneumatically connected, i.e., the pressure control unit 190 may comprise a portion in the cartridge 510 and a portion in the manifold 520. The bottom layer 730 may comprise channels to fluidically connect the elements mounted on the manifold. The manifold 520 may further comprise a plurality of inserts 740 (e.g., metal inserts) to fixedly mount elements on the manifold, for example to fix the selection valve 195, each of the one or more PCBs, sensor casing (see 785 in **figures 7D****),** sensor housing and/or the flowmeter 660.

**Figures 7C-D** present an example of the manifold 520 when other elements are mounted on the frame, in assembled and exploded views, respectively. An optional pressure release valve 780 and four (pressure) selection valves 195 (i.e., in total five valves as presented in **figure 7D****)** may be mounted on the frame. In examples where the pressure at the gas inlet 160 is provided by a pump (e.g., a piezoelectric pump), the apparatus 100 may comprise the pressure release valve 780 to release (i.e., regulate) pressure coming from the pump connected at the gas inlet 160 of the reservoir so that a decrease in pressure can be obtained rapidly. The pressure release valve 780 may not be used in examples when the pressure at the gas inlet 160 is provided by an external pressure controller. Further, the flowmeter 660 may be mounted on the frame, as well as liquid level sensors (i.e., liquid level detectors) 795. By way of example, a plurality (e.g., two) level sensor housings 785 may be installed via respective openings (i.e., apertures) 790 in the frame 705 of the manifold 520. Each level sensor housing may comprise one or more, preferably two, liquid level sensors 795, each dedicated to measure the level of liquid in one of the reservoirs, i.e., of the recirculation input reservoir, injection reservoir, recirculation output reservoir, and sampling reservoir. Each liquid level sensor 795 may be configured to detect at least if a liquid level in the reservoir is above a first threshold, and/or below a second threshold. The structure and mechanism related to the liquid level sensors are discussed in more detail below.

The manifold 520 may further comprise four magnetic coils 750 for example mounted on respective slots 755 on frame of the manifold 520 (or more precisely on the bottom layer 730 of the manifold 520) such that each magnetic coil 750 is located next to, for example under a respective reservoir when the cartridge 510 is mounted on the manifold 520. These magnetic coils 750 are discussed in more detail below.

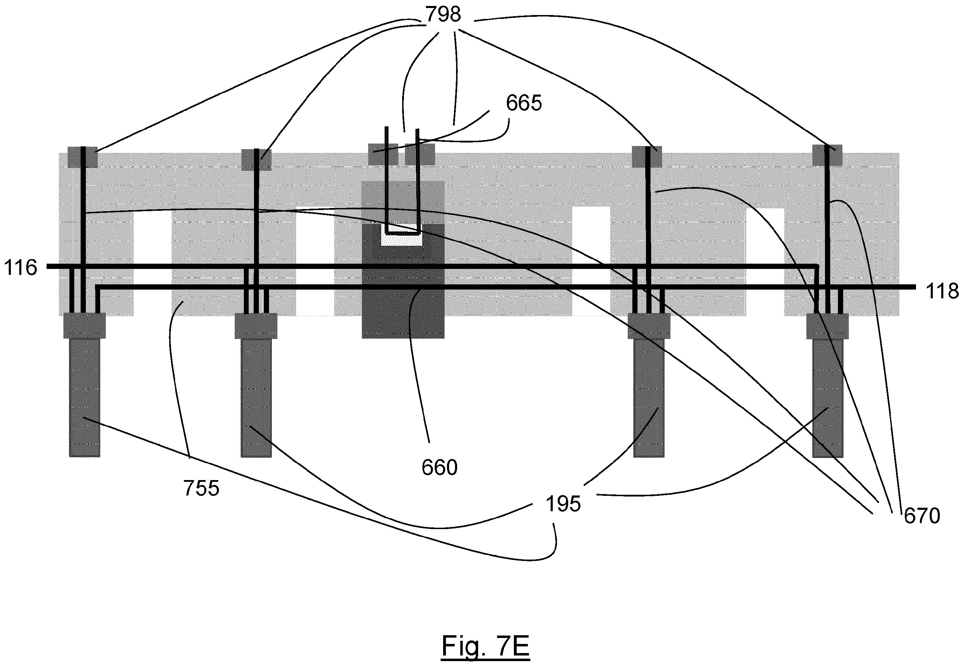

**Figure 7E** schematically shows the fluidic structure of the manifold 520. The manifold 520 may in particular comprise gas line portions 670 which are configured to be fluidically connected to respective gas line portions of the cartridge 510 and thus to the respective reservoirs. The fluidical connection of the liquid medium between the manifold 520 and the cartridge 510 is via the diversion ports 665 (see also **figure 6C****).**

The selection valves 195 are associated with these various gas line portions. Each selection valve 195 is fluidically connected to a first gas source 116 and a second gas source 118 via respective gas conduits.

The manifold may comprise at least one flowmeter 660, as well as channels leading to and from the flowmeter, configured to be fluidically connected to the channels in the cartridge. For example, the channels leading to and from the flowmeter 660 may form a portion of the first connection 112, or of the second connection 114, the other portion being in the cartridge 510, as described above.

Further, gaskets 798 (also see **figure 7C****)** may be provided for connecting respective lines or channels of the cartridge 510 and manifold 520. For example, four gaskets 798 may be installed on respective ports of the gas line portions 670 of the manifold, and two gaskets 798 may be installed on respective diversion ports 655 of the channels leading to and from the flowmeter.

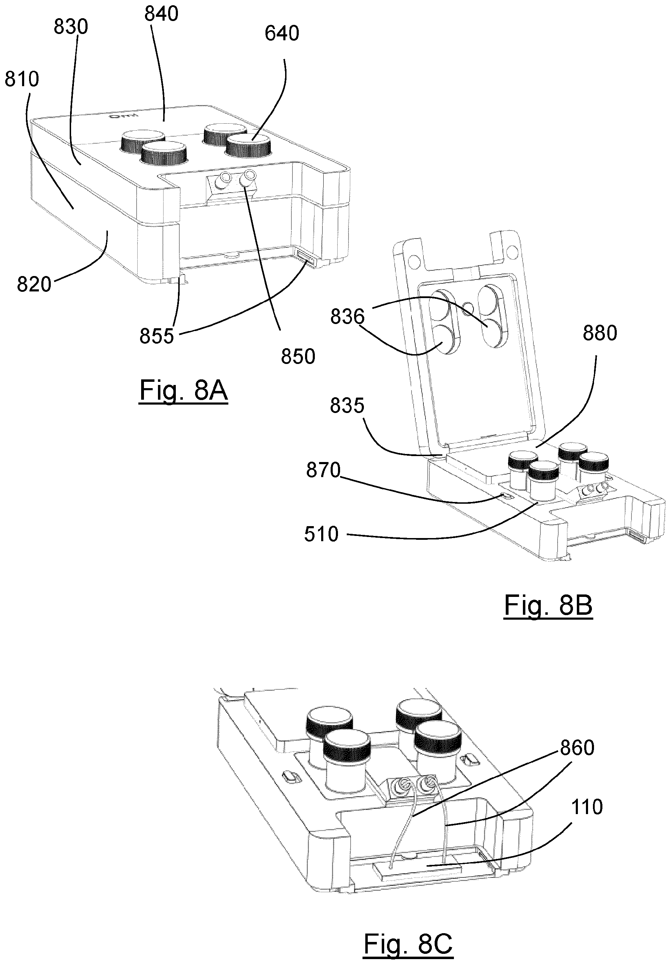

Another example of apparatus is presented in **figures 8A-E.** The apparatus 100 in this example further comprises a main housing 810 on which the cartridge 510 and the manifold 520 (which may be as described above) are mounted. The main housing 810 may comprise a chassis 820, and a housing cap 830 hinged (via a hinge 835) to the chassis 820 and configured to be opened or closed. **Figure 8A** displays the housing cap 830 when closed while **figure 8B** displays it when open. The housing cap 830 may further comprise a plurality of holes 836 adapted to receive respective reservoirs such that in particular the cap 640 of each reservoir is accessible when the housing cap 830 is closed.

The housing cap 830 may further comprise a clear window and a screen 840 (e.g., a tactile screen) below the clear window. The screen 840 may comprise a screen PCB 841 and be configured to display information regarding the status of apparatus, for example, being on/off or the flow level in each reservoir or a respective flow scenario. Instead of or in addition to a screen, any other user interface may also be employed. The screen may in a connection with a wireless PCB 842.

The chassis 820 may further comprises a locking bolt 870 to fix the mounting of the cartridge 510 and an internal cap 880 to access the pressure control unit 160. The locking mechanism 875 of the locking bolt is discussed later hereinbelow.

The chassis 820 may further comprise a holder 855 for the fluidic system 110. The holder 855 may hold the fluidic system 110 while being installed on the apparatus 100. The holder 855 may be in form of a groove (such that at least a part of the fluidic system 110 be inserted into the groove upon the installation), a protrusion (such that the protrusion is inserted into at least a part of the fluidic system 110), and/or a flat surface (such that at least a part of the fluidic system 110 lies on the flat surface upon the installation). The form of the holder may depend on the fluidic system 110. In examples, the holder 855 may be a removable part. Preferably a holder 855 of one form may be replaced (e.g., by a user) by a holder 855 in another form to be adapted to the fluidic system 100. In some applications, the form of the holder 855 may allow a fluidic system 110 to be connected to two or more apparatuses 100 at the same time. In such applications, the holder 855 may be installed on the chassis 820 of the two or more apparatuses 100 at the same time and to hold the fluidic system 100 connected to the two or more apparatuses. In such applications each of the connected two or more apparatuses 110 may be responsible for feeding a dedicated channel in the fluidic system 110.

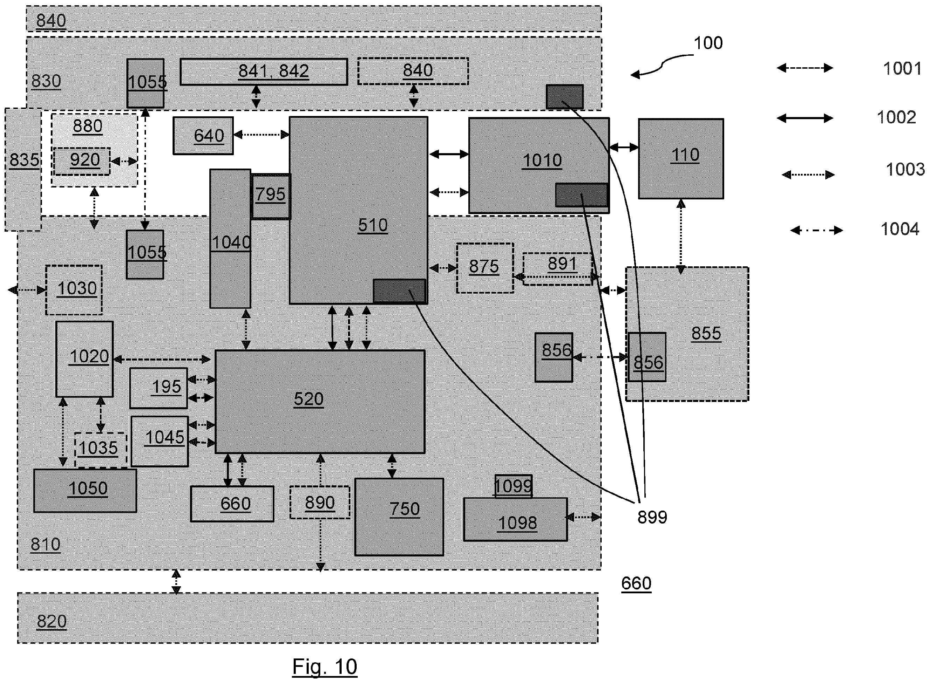

In examples, each of the chassis 820 and the holder 855 may further comprise a clamp magnet 856 allowing the holder 855 to be installed on the chassis 820 using the clamp magnet 856. The clamp magnet is presented in **figure 10****.**

The chassis 820 may further comprise an adapter 850 which allows the fluid connection of the apparatus to the fluidic system 110, e.g., a microfluidics chip. The adapter 850, in particular, may comprise two connection ports respectively fluidically connected to the first connection 112 and the second connection 114, thereby enabling the fluidical connection of the first connection 112 to an inlet of the fluidic system 110 and the fluidical connection of the second connection 114 to an outlet of the fluidic system 110. The connection of the adapter 850 to the first connection may be via the first port and to the second connection via the second port as discussed above. The connection of the adapter 850 to the fluidic system is presented in **figure 8C** where the fluidic system 110 is installed on the holder 855 of the chassis 820 and connected to the adapter 850 via (universal) intermediate tubing 860. Alternatively, the adapter 850 can provide a direct fluidic connection to the fluidic system without intermediate tubing 860. The adapter 850 may comprise one or more channels with controlled dimensions (not shown) configured to add extra hydrodynamic resistance in the connection to the fluidic system 110. This improves stability of the flow in the case of low resistance in the fluidic system 110 (e.g., low resistive microfluidics chip) and/or counter effects of hydrostatic pressure.

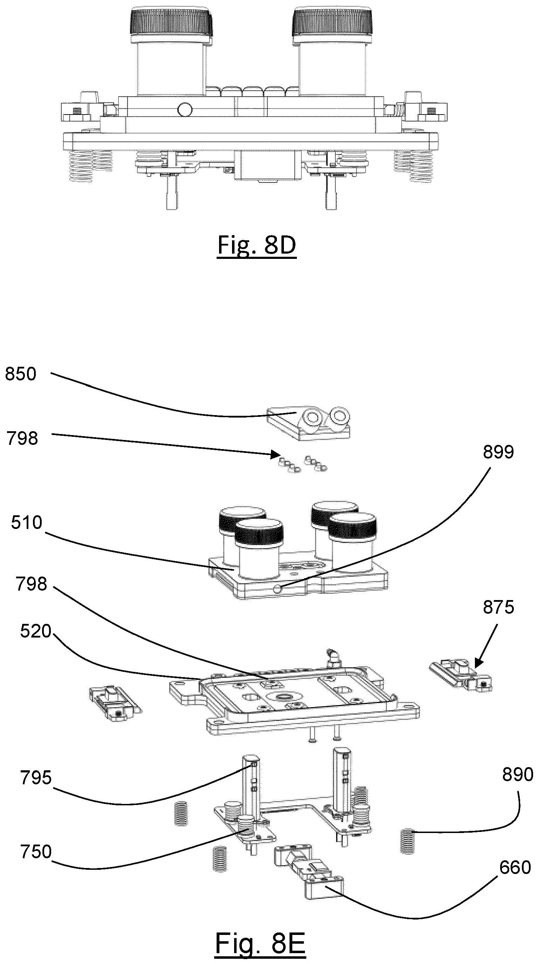

**Figures 8D** and **8E** present an example ensemble of cartridge 510 mounted on the manifold 520, in a side view and an exploded view, respectively. In addition to the elements already discussed, **figure 8E** displays a locking mechanism 875 (which may comprise a locking bolt 870 and a locking spring 891 (not shown)) and bottom springs 890 configured to support the manifold 520 relative to the chassis 820. The locking mechanism 875 (including the locking bolt 870 and the locking spring 891) and the bottom springs 890 are discussed in more detail below.

Furthermore, a magnet 899 may be mounted in the cartridge 510, the adapter 850, and/or top casing. Such a magnet may be used in cooperation with a respective Hall effect sensor (1099 in **figure 10****)** in the manifold 510 to determine if the cartridge 510 is correctly installed on the manifold 520, the adapter 850 (and the connected fluidic system 110 to the adapter) is installed on the manifold 820, and/or the top casing 830 is closed, for example before start of the feeding to the fluidic system 110 by the apparatus 100 to prevent leakage. In examples, the chassis 820 further comprises a PCB (1098 in **figure 10****)** with one or more, e.g. three, Hall effect sensors (1099 in **figure** 10) each being in cooperation with a magnet 899 mounted in the cartridge 510, the adapter 850, and top casing 830. Each Hall effect sensor is able to detect magnetic fields nearby and be used as presence sensors if a magnet 899 is placed in close contact to each sensor 1099, i.e., when the cartridge 510 is correctly installed on the manifold 520, the adapter 850 (and the connected fluidic system 110 to the adapter) is installed on the manifold 820, and/or the top casing 830 is closed.

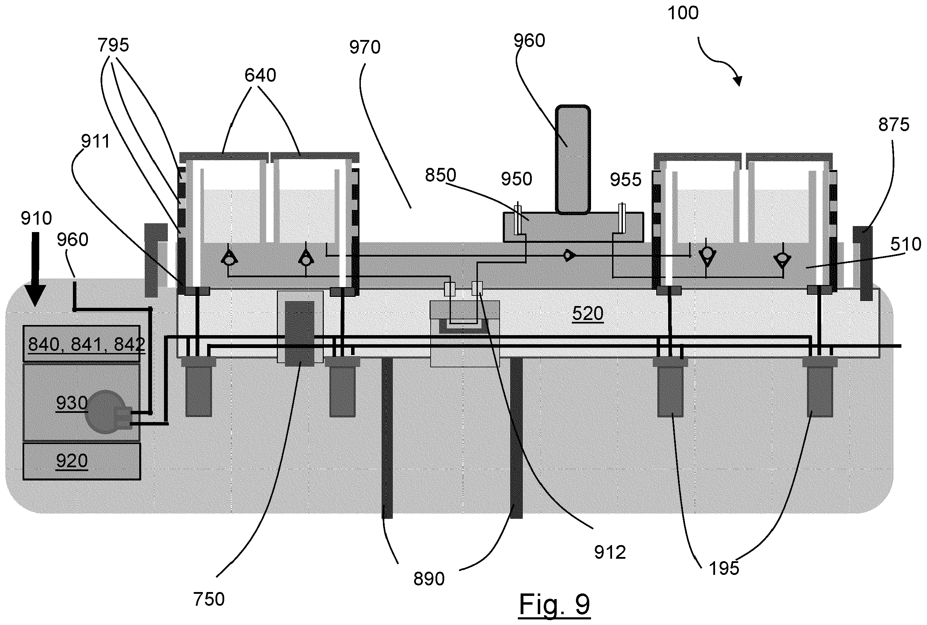

**Figure 9** presents a schematic cross-section of the apparatus. Not all elements already discussed above (and in particular **figures 6C** and **7E****)** are denoted in the **figure 9****.** In addition to the elements already discussed above, the drawing shows that the apparatus may comprise a first gas conduit fluidically connected to a first gas source 116 which may be for example the atmosphere; and a second gas conduit fluidically connected to a second gas source 118 which may be for example the outlet of a pumping device, which can be included in the apparatus. In this case, the inlet of the pumping device can be fluidically connected to the atmosphere. These gas conduits are fluidically connected to the selection valves 195. **Figure 9** further shows the fluidical connection between the perfusing channel (first connection 112 or second connection 114) and the flowmeter via contact connectors 912 comprising gaskets, as described elsewhere; and the fluidical (in this case, pneumatic) connection between the gas line portions of the cartridge 510 and the gas line portions of the manifold 520 via respective contact pressure connectors 911 (one for each reservoir) comprising gaskets, as described elsewhere. The apparatus 100 may comprise a pluggable air inlet 960. The apparatus 100 may also comprise a power source input 910, and a battery 920 for powering the various electrical components of the apparatus. Alternatively, the power source input 910 may be connected to an external power source. One or more control boards 930 may also be included in the apparatus 100 like a pressure control board. The apparatus 100 may also be fluidically connected to an inlet of the fluidic system 110 via 950 and to an outlet of the fluidic system 110 via 955. The apparatus 100 may further comprise a clampable high resistance module 970, as the adapter 850 described above. This clampable high resistance module 970 may comprise at least one channel configured to increase hydrodynamic resistance within the fluidic circuitry.

In this configuration, the housing cap 830 may be used for clamping the adapter 850 in its place, probably using a clamp magnet as discussed below in reference to **figure 10**.

**Figure 10** displays the global physical interconnections, i.e., pneumatic 1001, fluidic 1002, mechanical 1003, and magnetic 1004 of the apparatus 100. **Figure 10** also presents optional channels 1010 of the adapter 850 configured to add extra hydrodynamic resistance and the pump 1020 (e.g., a piezoelectric pump) to provide the pressure at the gas inlet as discussed above. In addition to the elements already discussed above, the apparatus 100 may therefore further comprise one or more of the following:
- a fan 1030 mechanically mounted on the main housing 810,
- a pressure sensor 1035 which connected to the control boards 930 as discussed above,
- PCBs 1040 of liquid level (e.g., optical) sensors mechanically mounted on the manifold 520,
- an emission valve 1045 pneumatically and mechanically connected to the manifold 520,
- a main PCB 1050 which is a part of the pressure control unit and in mechanical connection with the pump 1020, and
- a pair of clamp magnets 1055, one being configured to be mounted in the housing cap 830 and another one in the main housing 810 (the pair 1055 is configured to provide a magnetic clamping force to keep the housing cap 830 in a proximity of the main housing 810, i.e., to provide a resistive force against the housing cap to be opened), and
- springs 890 configured to mount the manifold 520 on the chassis 820, and the provide a locking capability of the locking mechanism 875 as discussed later below.

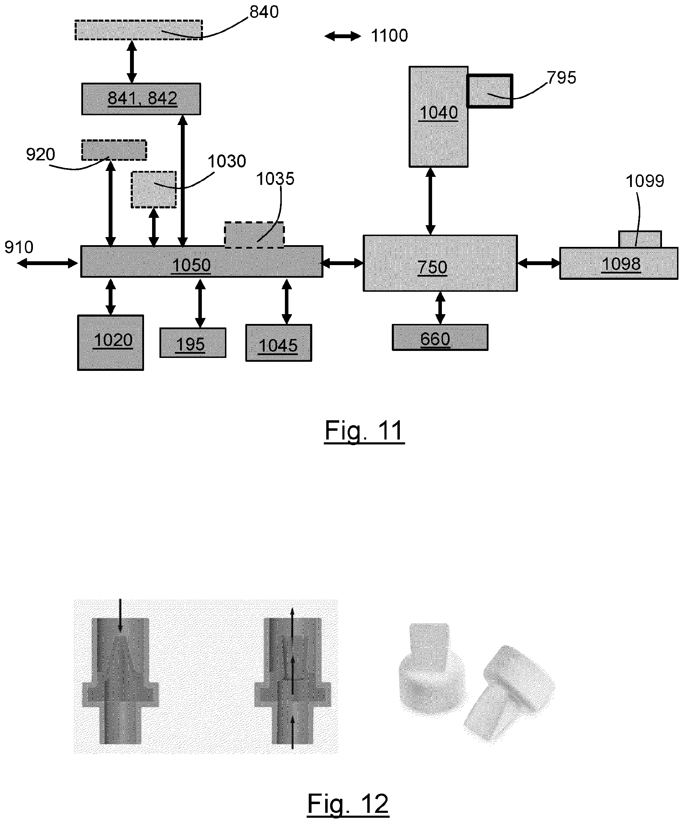

**Figure 11** presents the electrical interconnections 1100 in the apparatus 100. The electric power enters the apparatus from the power source input 910 and is supplied according to the interconnections depicted among the fan 1030, the battery 920, the pump 1020, different PCBs in the apparatus (1050, 1098, 1040, 841, and 842) and different sensors as discussed above.

### Pressure control and flow scenarios

In examples, as depicted in an example embodiment in **figure 3****,** the pressure control unit may allow or prevent a flow of liquid from or to each of reservoirs of the apparatus by adjusting the pressures in the reservoirs. In some embodiments, multiple (i.e., more than two) pressure levels may be applied in each reservoir. Each reservoir can be associated with a dedicated pressurizing mechanism (for example, a pump or a pressure controller). Alternatively, a finite number of pressure levels may be applied in each reservoir, by associating a dedicated selection valve with each reservoir and by fluidically connecting each selection valve to a number of gas sources at different pressure. The predetermined pressure levels can be the same for all reservoirs, or can differ between the reservoirs.

In the embodiments described in detail and illustrated above, only two (same) pressure levels are defined for all reservoirs, by connecting all selection valves 195 to a first gas source 116 and a second gas source 118, one of which can be at atmospheric pressure, and the other of which can be at a different pressure, preferably above atmospheric pressure.

Therefore, the pressure control unit 190 is configured for applying one of two pressure levels, i.e., "high" or "low" pressure level, at the gas inlet of each reservoir (and thus in each reservoir). The input pressure levels may equivalently be referred to as a control pressure or "Pcontrol", and an "exhaust" pressure or "Pexhaust". In examples, these two levels may vary over time. In other examples, these levels may remain constant over time. In some examples, Pexhaust may be fixed, for example to the atmospheric pressure, whereas the pressure control unit 190 controls Pcontrol. Pexhaust may be fixed by a direct connection of a port of the respective selection valve to the atmosphere. In some examples, Pcontrol is always greater or lower than Pexhaust, i.e., the two input pressure levels are not equal.

**Figure 4A** depicts an example of a selection valve 195 as used in the apparatus 100. In such examples, the selection valve 195 is a three ways-two positions (3/2) valve and comprises three ports: ports 401 and 402 are configured to be connected to a first gas source 116 and a second gas source 118, respectively, while the port 403, i.e., the common port or the selection port is configured to be connected to the gas inlet 160 of a reservoir. Such a selection port may fluidically connect either the port 401 or the port 402 to the selection port 403, thereby the pressure at port 403 is equal to either the pressure of the first gas source 116 or the second gas source 118, respectively (when pressure drops are neglected). The gas inlet 160 of each reservoir may be connected to the selection port 403 of the selection valve 195. The two other ports 401 and 402 of the selection valve 195 may be connected to the first gas source 116 and the second gas source 118, respectively thereby are respectively at Pcontrol and Pexhaust. Thereby, each valve controls the pressure in each reservoir to be either Pcontrol or Pexhaust.

In other examples, each selection valve dedicated to a reservoir may be composed of two two ways-two positions (2/2) valves each separately fluidically connecting a first gas source 116 and a second gas source 118 to the reservoir when open.

The pressure set in each reservoir by the pressure control unit 190 may determine how liquid medium flows in the apparatus and in the fluidic system. The pressure control unit 190 may notably effect flow of liquid medium in a perfusing step, in a refilling step, in an injection step and/or in a sampling step. In examples, the pressure control unit 190 may:
- in a perfusing step, control the gas pressure in the reservoirs so that liquid medium flows from the recirculation input reservoir 120 to the fluidic system 110 via the first connection 112, and is collected from the fluidic system 110 to the recirculation output reservoir 140 via the second connection 114;
- In a refilling step, control the gas pressure in the reservoirs so that liquid medium flows from the recirculation output reservoir 140 to the recirculation input reservoir 120; via the refill channel 185:
- in an injection step, control the gas pressure in the reservoirs so that liquid medium flows from the injection reservoir 130 to the fluidic system 110 via the first connection 112; in this case, liquid medium can be collected from the fluidic system 110 to the recirculation output reservoir 140 or to the sampling reservoir 150 via the second connection 114;
- in a sampling step, control the gas pressure in the reservoirs so that liquid flows from the fluidic system 110 via the second connection 114 to the sampling reservoir 150; in this case liquid medium can be supplied to the fluidic system 110 by the recirculation input reservoir 120 or by the injection reservoir 130.

In some variations, the perfusing step (and possible injection and/or sampling steps) may alternate with the refilling step.

The duration of each refilling step may be shorter than the duration of each perfusing step to avoid the fluidic system. In examples, the duration of a perfusion step may more than 1 minute, or more than 5 minute, or more than 10 minutes, or more than 30 minutes, or more than 1 hour, or more than 2 hours. It may be in a range between 1 minute and 30 days, or between 5 minutes and 15 days, or between 10 minutes and 7 days, or between 30 minutes and 1 day. The duration of a refilling step may be less than 5 minutes, or less than 1 minute, or less than 30 seconds, or less than 15 seconds. It may be in a range between 1 second and 5 minutes, or 2 seconds to 2 minutes, or 5 seconds to 1 minute, or 10 seconds to 30 seconds. This is especially advantageous in cases where the fluidic system 110 comprises an OOAC, as cells in such an OOAC need a persistent flow to survive and correctly grow. This can be possible by adjusting Pcontrol at a higher level during the refilling step, in order to achieve a higher flow rate. Alternatively, Pcontrol can have the same value in both steps but the flow rate may be higher in the refilling step because of the lower pressure drop between the reservoirs during this step. In examples, where the fluidic system comprises an OOAC, the duration of a refilling step depends on type of cells in the OOAC ang how long the cells are able to survive without nutrients and/or other chemicals (e.g., oxygen) during the refilling step.

When more than one recirculation reservoir pair is present in the apparatus, the refilling step and perfusing step can be carried out concurrently, namely liquid medium may flow from a first RI reservoir to the fluid system and then to a first RO reservoir (perfusing step), while at the same time liquid medium may flow from a second RO reservoir to a second RI reservoir (refilling step). At some point, the role of the reservoirs is switched, i.e., liquid medium may flow from the second RI reservoir to the fluid system and then to the second RO reservoir (perfusing step), while at the same time liquid medium may flow from the first RO reservoir to the first RI reservoir (refilling step). In this case, the flow of liquid within the fluidic system can be virtually uninterrupted. This can also be achieved similarly by using three or more recirculation reservoir pairs.

An injection step may be carried out simultaneously with part or all of a perfusion step. It may also be carried out simultaneously with a sampling step.

A sampling step may be carried out simultaneously with part or all of a perfusion step. It may also be carried out simultaneously with an injection step.

In other words, during an injection step, liquid medium from both the recirculation input reservoir 120 and the injection reservoir 130 may be simultaneously supplied to the fluidic system 110 (both flows being optionally mixed within the first connection 112). Alternatively, liquid medium may be supplied to the fluidic system 110 only from the injection reservoir 130. During an injection step, liquid medium flowing out of the fluidic system 110 may be collected in the recirculation output reservoir 140, or in the sampling reservoir 150, or in both.

Similarly, during a sampling step, liquid medium flowing out of the fluidic system 110 may be collected both in the recirculation output reservoir 140 and in the sampling reservoir 150. Alternatively, liquid medium from the fluidic system 110 may be collected only in the sampling reservoir 150. During a sampling step, liquid medium may be supplied to the fluid system by the recirculation input reservoir 110, or by the injection reservoir 120, or by both.

An injection step (preferably together with a sampling step) may be carried out simultaneously with a refilling step. In this case, liquid medium is supplied to the fluidic system 110 from the injection reservoir 130, while the refill takes place from the recirculation output reservoir 140 to the recirculation input reservoir 120. This also makes it possible to prevent or minimize interruption of flow within the fluidic system 110.

When more than one injection reservoirs and/or more than one sampling reservoirs are present, different injection steps may be sequentially or alternately carried out, i.e., liquid medium may flow to the fluidic system from different injection reservoirs at different points in time, and/or liquid medium may flow from the fluidic system to different sampling reservoirs at different points in time.

In examples of an apparatus 100 with four reservoirs, the gas pressure in each reservoir may be set at a value selected from a first pressure level Pcontrol and a second pressure Pexhaust. The table below shows various flow configurations depending on the pressure value in each reservoir I, RI, RO and O. In the table, P corresponds to Pcontrol and 0 corresponds to Pexhaust (reference level, preferably atmospheric pressure):

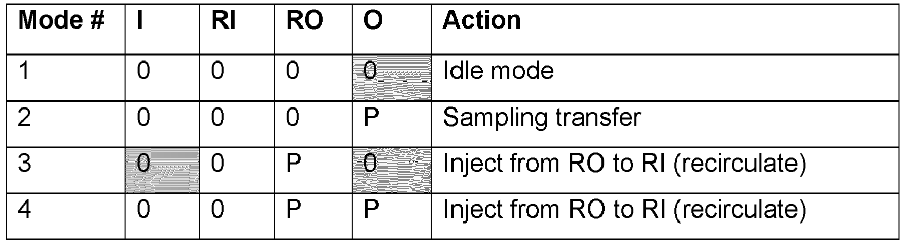

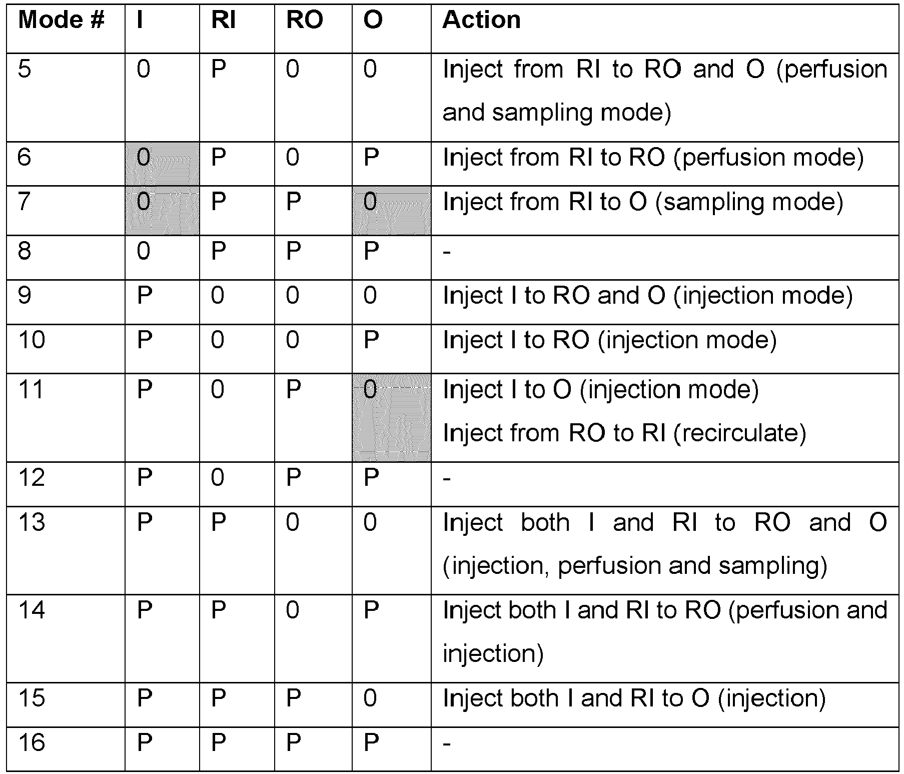

The various modes can be described as follows:
- Mode #1: All reservoirs are at Pexhaust pressure, i.e., the apparatus is in idle mode.
- Mode #2: All reservoirs but the reservoir O are at Pexhaust pressure. This mode may be used to transfer liquid medium from the sampling reservoir.
- Mode #3: All reservoirs but the reservoir RO are at Pexhaust pressure. In this mode, the apparatus quickly recirculates liquid medium to refill the reservoir RI from RO.
- Mode #4: The reservoirs RI and I are at Pexhaust pressure while the reservoirs RO and O are at Pcontrol. The apparatus quickly recirculates to refill the RI from RO.
- Mode #5: All reservoirs but the reservoir RI are at Pexhaust pressure. In this mode, the liquid medium is injected from RI to RO and O. The flowrate to the RO and O reservoirs may be different.
- Mode #6: The reservoirs RI and O are at Pcontrol while the reservoirs RO and I are at Pexhaust. This is a perfusion mode, and the liquid medium is injected from RI to RO.
- Mode #7: The reservoirs RI and RO are at Pcontrol while the reservoirs I and O are at Pexhaust. This is a sampling mode, and the liquid medium is injected from RI to O.
- Mode #8: All reservoirs but the reservoir I are at Pcontrol. There is no liquid flow. This mode should preferably be avoided as it may cause damage to the apparatus.
- Mode #9: All reservoirs but the reservoir I are at Pexhaust. In this mode, the liquid medium is injected from I to RO and O. The flowrate to the RO and O reservoirs may be different.
- Mode #10: The reservoirs RI and RO are at Pexhaust while the reservoirs I and O are at Pcontrol. This is an injection mode, and the liquid medium is injected from I to RO.
- Mode #11: The reservoirs I and RO are at Pcontrol while the reservoirs RI and O are at Pexhaust. In this mode, the liquid medium is injected from I to O (injection step) and quickly recirculated from RO to RI (refilling step).
- Mode #12: All reservoirs but the reservoir RI are at Pcontrol. There is no liquid flow. This mode should preferably be avoided as it may cause damage to the apparatus.
- Mode #13: The reservoirs RI and I are at Pcontrol while the reservoirs RO and O are at Pexhaust. In this mode, the liquid medium is injected from I and RI to RO and O (perfusion, injection and sampling).
- Mode #14: All reservoirs but the reservoir RO are at Pcontrol. In this mode, the liquid medium is injected from I and RI to RO. The flowrate from the RI and I reservoirs may be different. This is an injection and perfusion mode.
- Mode #15: All reservoirs but the reservoir O are at Pcontrol. In this mode, the liquid medium is injected from I and RI to O. The flowrate from the RI and I reservoirs may be different. This is an injection mode.
- Mode #16: All reservoirs are at Pcontrol, i.e., the apparatus is in idle mode.

By the "sampling transfer" above is meant examples in which the reservoir O further comprises a liquid outlet (e.g., a piece of tubing coming through the housing cap 830) connected to an external reservoir/sampler. The outlet may include a fluidic valve to allow the flow out of the reservoir O. Such an outlet makes it possible to transfer the content of sampled liquid at the reservoir O during the sampling mode to an external reservoir (e.g., automatically). The external reservoir may be a collection well in a multiple-well (e.g., 96-well) plate reservoir. In examples, the multiple-well plate may be part of a robot that can move the plate reservoir to change the collection well fluidically connected to the reservoir O. The sampling transfer step does not effect on the flow in the apparatus 100.

Furthermore, the gray boxes in the above table denote that pipetting is possible in the corresponding reservoir. Indeed, when a reservoir is at Pexhaust and if Pexhaust is the atmospheric pressure, the cap of the reservoir may be opened to pipet liquid and/or to grant access to the reservoir by the user without affecting the functioning of the apparatus.

### Connection and locking mechanisms

Now examples of connection and locking mechanisms in the apparatus are discussed.

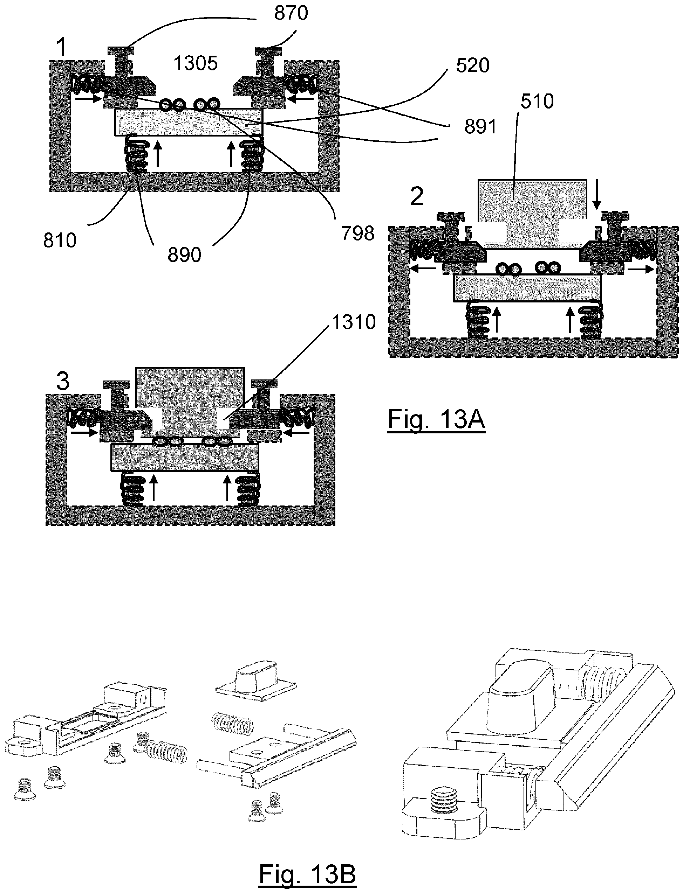

Firstly, the operation of the cartridge locking mechanism of the apparatus 100 is discussed with reference to **figures 13A-C**. The apparatus comprises a manifold and a cartridge as described above.

A locking mechanism using bolts is presented in **figure 13A****.** It makes it possible to lock the cartridge 510 relative to the manifold 520 thus ensuring a proper fluidic connection between these parts.

In part 1) of **figure 13A****,** no cartridge is mounted on the manifold, i.e., the manifold 520 is in a default position. In this position, the manifold 520 is in contact with the main housing 810. Preferably, bottom springs 890 as described above may push the manifold 520 away from a bottom wall of the main housing 510 into contact with an upper part of the housing. The black arrows show the direction of the force applied by the bottom springs 890 and/or the locking springs 891.

In part 2) of **figure 13A****,** the cartridge is inserted. It may be pushed towards the manifold (and be guided vertically) through an opening 1305 in the main housing 810. At least one bolt 870, preferably at least two bolts 870, may be present on the sides of this opening 1305. They may have a tapered portion and may be slidably mounted on the main housing 810, so as to be laterally movable and so as to assume a retracted position (closer to the periphery of the opening) and an extended position (closer to the center of the opening). A locking spring 891 may be associated with each bolt 870 in order to bias it towards the extended position. The bolts 870 may thus be laterally pushed towards the periphery of the opening 1305 when the cartridge 510 is inserted. Alternatively or additionally to the bolts 870 having a tapered portion, the cartridge 510 itself may have one or more tapered portions.

In part 3) of **figure 13A****,** the cartridge is fully inserted into the opening of the housing and cannot further be pushed down due to the contact of the cartridge 510 with the bolts 870, and the bolts 870 may biased back return to their extended position by the respective locking springs 891. In this extended position, they may lock, i.e., block any movement of the cartridge 510, due to engagement of the bolts 870 with respective recesses 1310 on the cartridge 510. The recesses may be part of a single peripheral groove around the cartridge or they may be separate recesses. In this position, gaskets 798 may be compressed between the manifold 520 and the locked cartridge 510 because the bottom springs 890 force the manifold 520 towards the cartridge 510, and the cartridge 510 is blocked in the main housing 810. In this position there may thus be a leak-tight connection between the cartridge 510 and the manifold 520, so that liquid and gas can flow from the manifold 520 to the cartridge 520 or vice versa, up to a certain maximum pressure value which depends on nature of the gaskets 798 and on the stiffness of the bottom springs 890.

**Figure 13B** shows an assembled view and an exploded view of said locking mechanism.

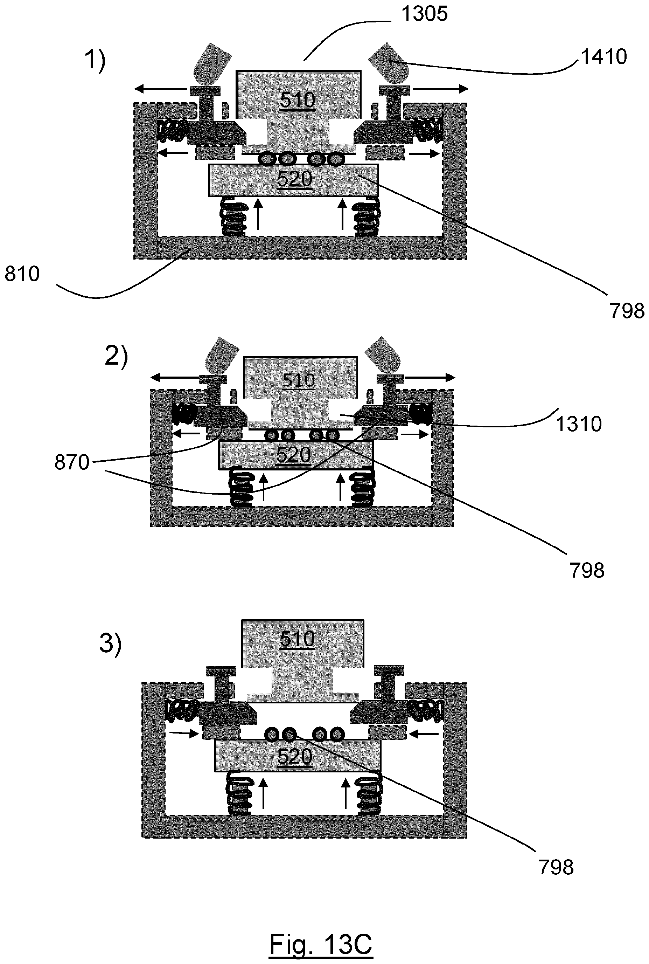

The unlocking operation is presented in **figure 13C**.

In part 1) of **figure 13C****,** a user may use her/his fingers 1410 to force the or each bolt 870 into their retracted position by pushing them towards the periphery of the opening 1305 against the biasing force of the respective locking springs 891.

In part 2) of **figure 13C****,** the user continues to press on the bolt(s) 870 until they disengage from the recesses 1310 or groove so that the cartridge 510 is unlocked. The cartridge 510 may then be ejected due to the bottom springs 890 which push the manifold 520 toward the upper part of the main housing 810 and therefore towards the opening 1305.

In part 3) of **figure 13C** the user stops pressing on the bolt(s) 870. Thereby the bolt(s) 870 go back to the extended position owing to the biasing force of their respective locking springs 891. The cartridge 510 can then be removed from the main housing 810. The black arrows show the direction of the force applied by the bottom springs 890, the locking springs 891, and/or the finger 1410.

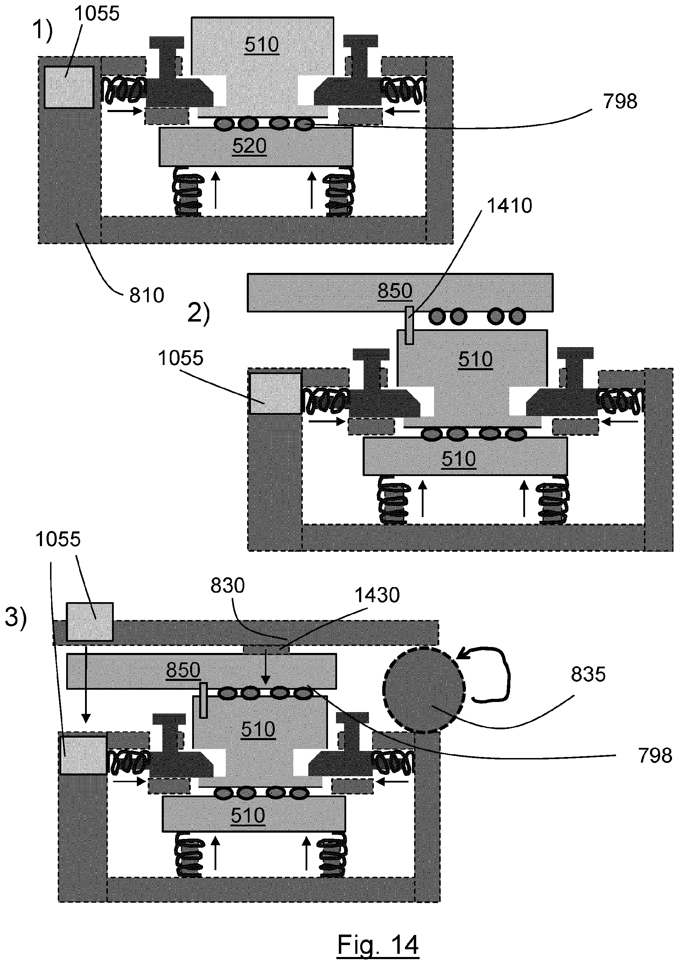

Secondly, the operation of a (fluidic) adapter locking mechanism of the apparatus 100 is discussed in reference to **figures 14****.** It makes it possible to lock the adapter 850 (as described above) relative to the cartridge 510 and manifold 520, thus ensuring a proper fluidic connection between these parts.

In part 1) of **figure 14****,** the cartridge 510 is locked with the manifold 520, and the adapter 850 is not present. The main housing 810 is open.

In part 2) of **figure 14****,** the adapter 850 is aligned with the cartridge 510, for example thanks to an alignment pin/structure 1410.

In part 3) of **figure 14****,** the housing cap 830 may rotate around its hinge 835 to its closed position (in the direction of the black arrow). By way of example, (a pair of) clamp magnets 1055 may be provided on the housing cap 830 and on a main housing 810, so that the housing cap 830 presses against the adapter 850. Further gaskets 798 may be provided on the cartridge 510 and/or on the adapter 859, to ensure a leak-tight fluidic connection between the adapter 850 and the cartridge 510, in view of the pressure exerted by the housing cap 830 on the adapter 850. Preferentially, this pressure is exerted by a protruding portion 1430 of the housing cap 830 which comes into contact with the adapter 850. Preferentially, this protruding portion 1430 contacts the adapter 850 approximately at the barycenter of all gaskets 798. It is also possible to provide several protruding portions. Instead of clamp magnets 1055, a mechanical locking system (for example including a bolt and spring) may be provided. The black arrows show the direction of the force applied by the bottom springs 890, the locking springs 891, and/or the pressure exerted by the protruding portion 1430.

### Liquid level detection

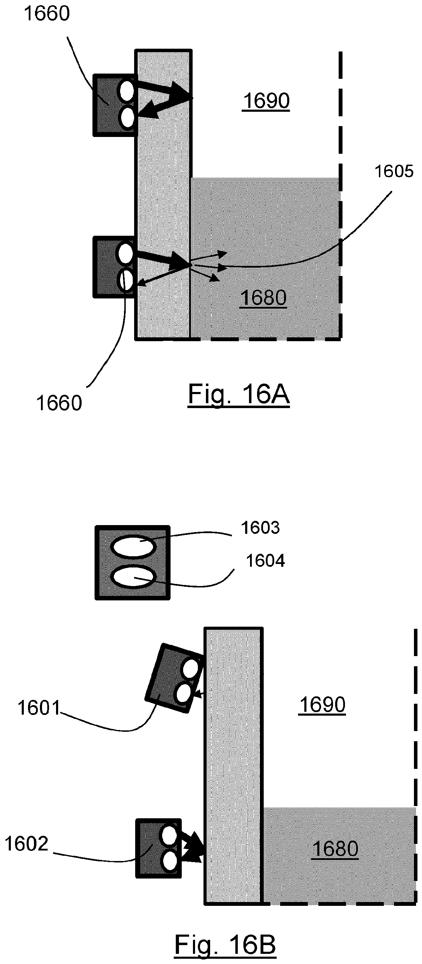

Making reference to **figures 16A-16E****,** the liquid level sensors 795 used in the apparatus 100 may be optical units comprising, in a single component, a LED and a photoreceptor, for example an infrared LED and an infrared photoreceptor. The liquid level sensors 795 may be used to detect the presence or absence of a liquid in a reservoir.

In examples, each liquid level sensor may have an emitting cell 1603 (e.g. al LED) and a receptor cell 1604. The emitting cell 1603 may emit an optical signal towards the reservoir and receive the reflection of the emitted signal by the receptor cell 1604. The reservoir is preferably made of a material having a refractive index close to the refractive index of water. Polycarbonate is an example of such a material. Due to higher light scattering 1605 in liquid 1680 compared to gas 1690, the liquid level sensor may detect a level of liquid in the reservoir. More precisely, if (the receptor cell 1604 of) a liquid level sensor 759 receives high signal (i.e., a signal with high level of energy) as the reflection of the emitted signal to the reservoir, it means that the signal has emitted into and been reflected from the gas 1690 in the reservoir, thereby the liquid level in the reservoir is below the level of said level sensor 795. Similarly, if (the receptor cell 1604 of) a liquid level sensor 759 receives low signal (i.e., a signal with low level of energy) as the reflection of the emitted signal to the reservoir, it means that the signal has emitted into and been reflected from the liquid 1680 in the reservoir, thereby the liquid level in the reservoir is at least equal to the level of said level sensor 795.

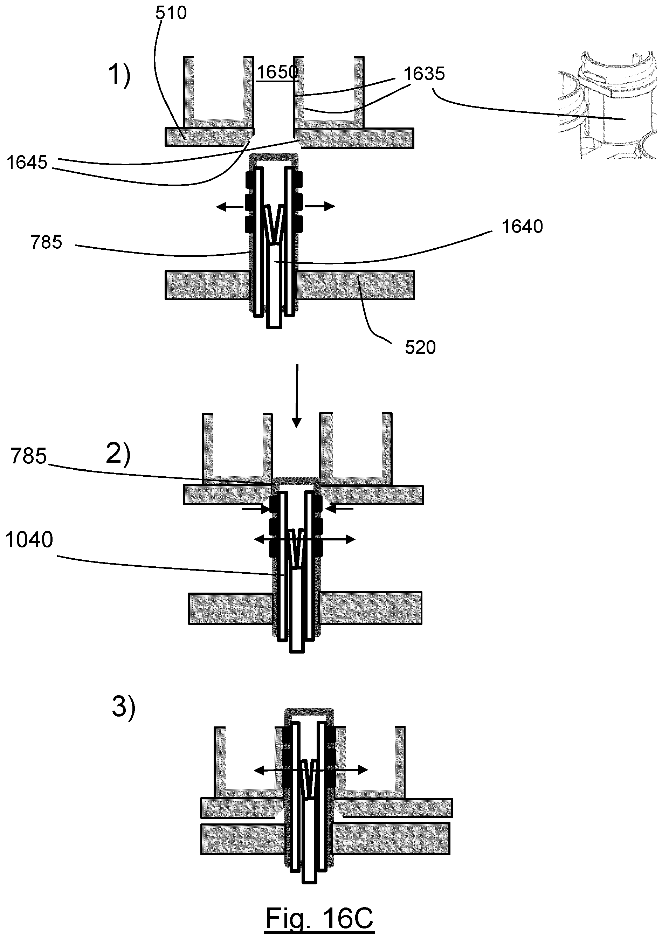

Each reservoir may be provided with more than one liquid level sensors, in order to detect the presence or absence of liquid at various heights in the reservoir. For example, each reservoir may be provided with two liquid level sensors, for detecting a High liquid level (H) and a Low liquid level (L). An example of a reservoir with two liquid level sensors H 1660, and L 1670 is presented in **figure 16A****.** Or each reservoir may be provided with three liquid level sensors for detecting a High liquid level (H), a Middle liquid level (M) and a Low liquid level (L). An example of a reservoir with three liquid level sensors H 1660, M 1665, and L 1670 is presented in **figures 16C-16D****.** More than three liquid level sensors could also be provided.

When the apparatus comprises a cartridge-manifold structure as described above, the liquid level sensors may be mounted on a vertically disposed PCB 1040 fixed to the manifold 520 and configured to be positioned along a reservoir wall 1610 when the cartridge 510 (including the mounted reservoirs) is mounted on the manifold 520.

In examples, these PCBs 1040 may be grouped together, for example two by two (i.e., two PCBs 1040 each for one reservoir), in respective level sensor housings (or casing) 785 (see **figure 7D****)** fixed on the manifold 520. The liquid level sensors may protrude from respective openings in each casing 785. Further, at least a portion of a wall of each reservoir and a portion of each casing 758 may be flat. These flat surfaces 1635 improve the accuracy of the sensor by avoiding tilted sensors as depicted in **figure 16B****.** A tilted sensor 1601 may lead to crosstalk (i.e., the receptor cell 1604 of a liquid level sensor receives signals from another sensor) or false positive.

Further in such examples, inside the level sensor housing 785 a spring, such as a Y-spring 1640, may be provided in a central position, i.e., between the PCBs 1040, so that the sensors are pressed on the flat surface 1635 on their respective reservoir. This improves accuracy by avoiding any airgap between a sensor and the reservoir wall as depicted in **figure 16B****.** An airgap 1602 may lead to false negative for the sensor, or no signal at the receptor if the gap is too large.

The liquid level sensor insertion and positioning in an embodiment are now discussed in reference to **figure 16C****.**

In part 1 of **figure 16C****,** the insertion of the cartridge 510 is started. The cartridge 510 comprises spacings 1650 between respective reservoirs in which respective level sensor housings 785 can be introduced. The bottom of the cartridge 510 may comprise beveled portions 1645 to facilitate the insertion of level sensor housings 785 in a respective spacing 1650.

In part 2 of **figure 16C** the cartridge 510 is pushed so that each level sensor housings 785 penetrates a respective spacing 1650. When each liquid level sensor 795 contacts a reservoir wall, it is pushed inwards in its level sensor housings 785, and the spring biases the respective PCB 1040 and thus the sensor 795 against the reservoir wall 1635.

In part 3 of **figure 16C****,** the cartridge 510 is fully mounted on the manifold 520, and each casing 785 is fully inserted in the spacing 1650 such that all sensors 795 are properly aligned and in contact with the reservoir wall without air gap.

The black arrows show outward forces exerted by the Y-spring 1640 on the level sensor housing 785, and/or by the spacing 1650 on the level sensor housing 785, and/or a downward force exerted on the cartridge 510 to mount it on the manifold 520.

This insertion process can be repeated multiple times thanks to the spring.

The liquid level sensors 795 may be used to automatically switch between perfusion and refill modes. For example, when the recirculation input reservoir is detected as having a low liquid level or the recirculation output reservoir is detected as having a high liquid level, the pressure control unit may launch a refilling step as discussed above. Furthermore, when the recirculation output reservoir is detected as having a low liquid level or the recirculation input reservoir is detected as having a high liquid level, the pressure control unit may launch a perfusion step as discussed above.

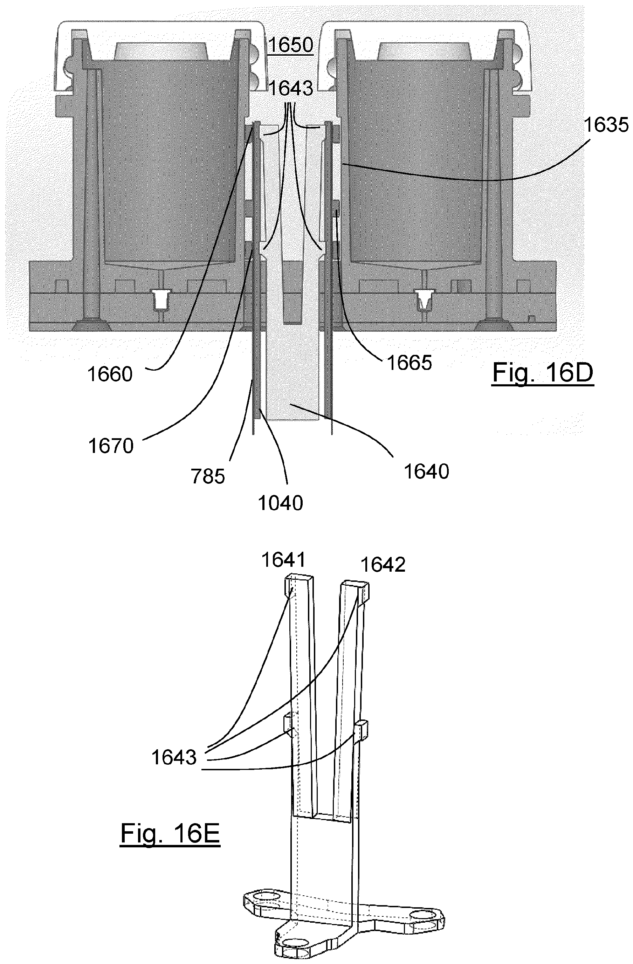

**Figure 16D** shows a cut view of the level sensor housings 785 inserted into the spacing 1650 and **figure 16E** is a 3D view of the Y-spring 1640 showing the locations of protrusions 1643 on the branches 1641 and 1642. **Figure 16D** further shows a mechanism that the Y-spring 1640 presses the liquid level sensors 795 on the flat surface 1635 on their respective reservoir by pressing the level sensor housings 785 via protrusions 1643. The number of protrusions 1643 may be two or more and are placed on each of two branches 1641 and 1642 of the Y-spring 1640.

In examples, any other type of liquid level sensors other than the type discussed above may be used. For example, the liquid level sensor may be a linear Charge-Coupled Device (CCD). Such CCD sensors may provide a high resolutive level sensing which is almost continuous.

Furthermore, the level detection methods discussed here may be additionally use to recalibrate the flowmeter 660 in case of drift. In such case, by knowing the distance (i.e., height differences along the reservoir) between two or more liquid level sensors 795 and the reservoir cross sectional area, a mean flowrate to fill the volume between two liquid level sensors may be computed. By a comparison of the computed mean flowrate and the rate detected by the flowmeter 660, the flowmeter 660 may be recalibrated.

### Magnetic agitation mechanism

The apparatus 100 may comprise magnetic coils 750 configured to perform magnetic agitation in the reservoirs. When the apparatus 100 comprises a manifold 520 and a cartridge 510, these coils 750 may be mounted on the manifold 520. For example, they may be mounted on respective slots 755 on the manifold 520 such that each coil 750 is located under a respective reservoir when the cartridge 510 is mounted on the manifold 520. A magnetic agitator (i.e., a magnetic barrel) may be placed in each reservoir. By activating each magnetic coil, a magnetic field is generated in each reservoir; as a result the magnetic agitator in the respective reservoir moves, for example rotates around a rotation axis for e.g., oriented vertically. The agitation may improve performance by controlling the uniformity of the liquid medium.

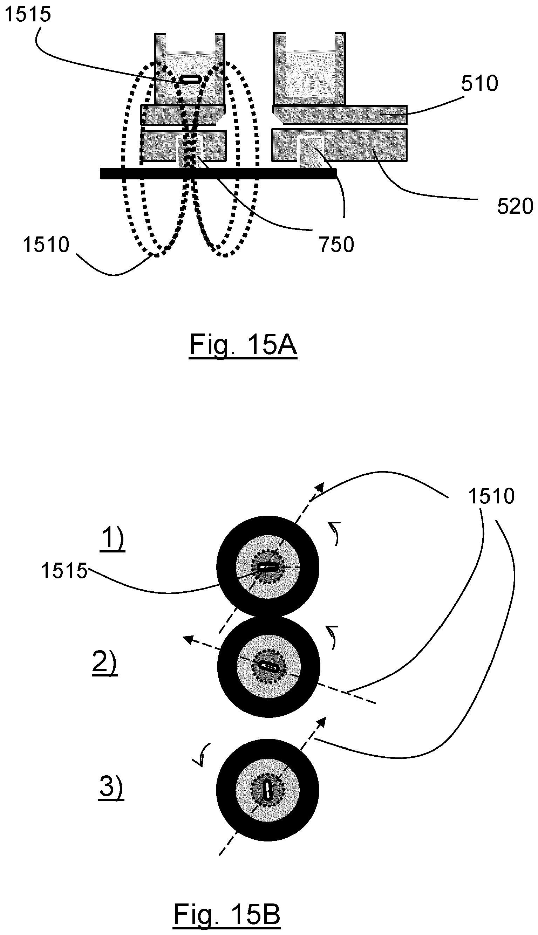

The mechanism of magnetic agitation is now discussed in reference to **figures 15A-B.**

When electric current flows through a magnetic coil 750, for example, upon a control signal from a control unit (for example from the pressure control unit 190), a magnetic field 1510 is generated around the magnetic coil 750, and a magnetic barrel 1515 placed in the reservoir moves to align with the magnetic field 1510. By turning the field on and off at a certain frequency, the magnetic barrel 1515 vibrates or spins due to inertia.

**Figure 15B** shows a top view of different steps of stirring by the magnetic barrel 1515. The (direction of the) magnetic field 1510 is presented by the dashed arrow 1510. In step 1), the electric current in the magnetic coil 750 and the magnetic field 1510 are turned on, thus the barrel 1515 rotates (along the solid black arrow) to align to the magnetic field 1510. In step 2), the electric current in the coil 750 and the magnetic field 1510 are turned off and the barrel 1515 keeps rotating (along the solid black arrow) due to inertia. In step 3), the electric current in the coil 750 and the magnetic field 1510 are turned on again which adds energy to the barrel 1515 to continue the rotation (along the solid black arrow).

Magnetic agitation may be turned on or off independently in each reservoir.

## Claims

1. An apparatus (100) for feeding a fluidic system (110) with liquid medium, the apparatus (100) comprising:
- a recirculation input reservoir (120) and an injection reservoir (130), each comprising:
∘ at least one gas inlet (160), and
∘ at least one liquid outlet (165) equipped with a check valve (175) configured to allow a flow of liquid out of the reservoir;
- a recirculation output reservoir (140) and a sampling reservoir (150) each comprising:
∘ at least one gas inlet (160), and
∘ at least one liquid inlet (170) equipped with a check valve (180) configured to allow a flow of liquid into the reservoir;
- a refill channel (185) fluidically connecting at least the recirculation input reservoir (120) and the recirculation output reservoir (140), said channel being equipped with at least one check valve (186) configured to allow a flow of liquid medium from the recirculation output reservoir (140) to the recirculation input reservoir (120);
- a pressure control unit (190) comprising gas lines connected to the gas inlets (160) of the recirculation input reservoir, injection reservoir, recirculation output reservoir, and sampling reservoir and configured to control gas pressure in the recirculation input reservoir, injection reservoir, recirculation output reservoir, and sampling reservoir; and
- a first connection (112) and a second connection (114) each configured to be respectively connected to an inlet and to an outlet of the fluidic system (110);
wherein
- the recirculation input reservoir (120) and the injection reservoir (130) are configured for being fluidically connected to the fluidic system (110) via the first connection (112); and
- the recirculation output reservoir (140) and the sampling reservoir (150) are configured for being fluidically connected to the fluidic system (110) via the second connection (114).

2. The apparatus according to claim 1, wherein the pressure control unit is configured to set gas pressure in the recirculation input reservoir, injection reservoir, recirculation output reservoir, and sampling reservoir at a value selected from a first pressure level and a second pressure level, at least one of the first pressure level and second pressure level being preferably the atmosphere pressure level.

3. The apparatus according to any one of claims 1 to 2, wherein the pressure control unit comprises four selection valves on the gas lines respectively connected to the gas inlets of the recirculation input reservoir, injection reservoir, recirculation output reservoir and sampling reservoir.

4. The apparatus according to claim 3, wherein each selection valve is fluidically connected to a first gas source at the first pressure level and to a second gas source at the second pressure level, and wherein preferably at least one of the first gas source 116 and second gas source 118 is the outlet of a pumping device.

5. The apparatus according to any one of claims 3 or 4, wherein each selection valve is a three ways - two positions valve.

6. The apparatus according to any one of claims 1 to 5, wherein each of the recirculation input reservoir, injection reservoir, recirculation output reservoir, and sampling reservoir is equipped with one or more liquid level sensor.

7. The apparatus according to claim 6, wherein each liquid level sensor is configured to detect at least if a liquid level in the reservoir is above or below a threshold level.

8. The apparatus according to any one of claims 1 to 7, comprising a cartridge and a manifold, the cartridge being configured to be removably mounted on the manifold, wherein
- the recirculation input reservoir, injection reservoir, recirculation output reservoir, and sampling reservoir are in the cartridge; and
- the pressure control unit is in the manifold.

9. The apparatus according to claim 8, wherein:
- the manifold further comprises a printed circuit board (PCB) embedding comprising a plurality of magnetic coils, each magnetic coil being positioned so as to generate a magnetic field in a respective reservoir when the cartridge is mounted on the manifold; and/or
- the first connection (112) and/or the second connection (114) comprises a channel, a portion of which is in the cartridge and a portion of which is in the manifold, the portion in the manifold comprising a flowmeter; and/or
- the manifold comprises a plurality of liquid level sensors, each liquid level sensor positioned so as to detect a liquid level in a respective reservoir when the cartridge is mounted on the manifold.

10. An assembly comprising the apparatus (100) of any one of claims 1 to 9 and a fluidic system (110), the fluidic system (110) being fluidically connected to the first connection (112) and the second connection (114) of the apparatus (100), preferably via an adapter (850).

11. A method of feeding a fluidic system (110) with liquid medium, wherein said fluidic system (110) is fluidically connected to the first connection (112) and the second connection (114) of the apparatus (100) according to any of claims 1 to 13, the method comprising supplying liquid medium to the fluidic system (110) via the first connection (112) and collecting liquid medium from the fluidic system via the second connection (114).

12. The method according to claim 11, comprising
- a perfusing step, wherein the gas pressure in the reservoirs is adjusted so as to expel liquid medium from the recirculation input reservoir (120) to the fluidic system (110) via the first connection (112), and to collect liquid medium from the fluidic system (110) to the recirculation output reservoir (140) via the second connection (114); and/or
- a refilling step, wherein the gas pressure in the reservoirs is adjusted so as to make liquid medium flow from the recirculation output reservoir (140) to the recirculation input reservoir (120) via the refill channel (185).

13. The method according to any one of claims 11 or 12, comprising:
- an injection step, wherein the gas pressure in the reservoirs is adjusted so as to expel liquid medium from the second reservoir (130) to the fluidic system (110) via the first connection (112); and/or
- a sampling step, wherein the gas pressure in the reservoirs is adjusted so as to collect liquid medium from the fluidic system via the second connection (114) to the sampling reservoir (150).

14. The method according to any one of claims 11 to 13, wherein the liquid medium is a culture medium and wherein the fluidic system comprises living cells.

15. A non-transitory computer readable storage medium having stored thereon instructions that, when executed, cause at least one control unit (160) to carry out the method of any one of claims 11 to 14.
